# EUROPÄISCHE PATENTANMELDUNG

(11) **EP 4 821 520 A1**
(43) Veröffentlichungstag der Anmeldung: **14.10.2026**
(21) Anmeldenummer: 25169751.2
(22) Anmeldetag: 10.04.2025
(51) Int. Cl.: A61M 5/168, A61M 5/142, A61M 25/00, A61M 25/10, A61M 5/145

(54) **VORRICHTUNG ZUR KONTROLLIERTEN UND DOSIERTEN ABGABE VON WIRKSTOFFFLÜSSIGKEITEN IM KÖRPERINNEREN**

(71) Anmelder: MeKo Manufacturing e.K., 31157 Sarstedt (DE)
(72) Erfinder: MEYER-KOBBE, Clemens, 31157 Sarstedt (DE)
(74) Vertreter: Arth, Hans-Lothar

(57) **Zusammenfassung**

Die vorliegende Erfindung betrifft eine Vorrichtung (1) zur dosierten Freisetzung einer Wirkstoffflüssigkeit (20) über eine Wirkstoffflüssigkeitsabgabeeinheit (50) im Körperinneren umfassend eine Pumpeinheit (2) mit angeschlossenem schlauchartigen Zuführsystem (40), mindestens eine Drossel (7) zur Begrenzung des Volumenstroms verbunden mit einem Druckaufbau im Zuführsystem, einen Drucksensor (3) zur Bestimmung des durch die Pumpeinheit erzeugten Drucks, einen Zeitgeber (4), der ausgelegt ist, einen Zeitraum, in dem die Wirkstoffflüssigkeit freigesetzt wird, zu erfassen, eine Recheneinheit (5), die konfiguriert ist, das von der mindestens einen Drossel (7) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Drosseltyp (7) und für diese Wirkstoffflüssigkeit (20) zu berechnen, und eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen.

## Beschreibung

Bei der Verabreichung von Medikamenten sind die gezielte Zuführung und die Dosierung der Wirkstoffe von elementarer Bedeutung. Zur Behandlung von körperinneren Organen ist allerdings die lokale Abgabe der Wirkstoffe, möglichst nur am gewünschten Wirkort, selten möglich, wodurch die Wirkstoffdosierung mit großen Unsicherheiten verbunden ist.

Die Behandlung von körperinneren Organen erfolgt im Regelfall durch Tabletten, dem intravenösen Spritzen von Medikamenten oder der Gabe von Infusionslösungen. Die Wirkung derartiger Medikamentenzufuhren ist systemisch, betrifft also den ganzen Organismus. Nur ein Bruchteil der Medikamentendosis gelangt zur gewünschten Wirkstätte, der Rest verteilt sich im ganzen Körper. Daher unterliegt die Medikamentendosierung mengenmäßig und von der Verabreichungsdauer Beschränkungen, um Kollateralschäden anderer Organe zu verhindern.

Eine effektive, spezifische Behandlung von Organen bzw. Krankheiten (z.B. Infektionen, Tumorerkrankungen) mit Medikamenten sind daher enge Grenzen gesetzt. Eine wirksame Medikamentenverabreichung (z.B. von Antibiotika) lässt sich häufig nicht erzielen oder nicht über einen längeren Zeitraum durchführen.

Idealerweise sollte die Medikamentenabgabe lokal, am gewünschten Wirkort erfolgen, um negative systemische Wirkungen auf den Organismus zu vermeiden. Hierzu existieren katheterbasierte Verfahren, um z.B. mit gebohrten Kathetern oder Kathetern mit ausfahrbaren Injektionsnadeln lokal Wirkstoffe, die in einer Flüssigkeit gelöst sind, abzugeben.

Die Schwierigkeit besteht dabei darin, die Flüssigkeitsmenge und damit die abgegebene Medikamentenmenge vor Ort zu kontrollieren bzw. genau zu bestimmen. Dies umso mehr, wenn kleine Medikamentenmengen von wenigen Millilitern oder sogar unter einem Milliliter lokal verabreicht werden sollen, z.B. direkt in der Niere oder Leber etc. oder in einem Blutgefäß z.B. zur Lyse eines Thrombus bei einem Schlaganfall.

Folgende Herausforderungen bestehen:
a) Die präzise Messung des abgegebenen Flüssigkeitsvolumens ist aufwändig und insbesondere bei kleinen Mengen nur mit hohem technischem Aufwand möglich. Im klinischen Alltag sind derartige Messeinrichtungen, die kalibriert werden müssten, kaum anwendbar. Die Durchflussmessgeräte müssen zudem steril sein.
b) Selbst wenn die Messapparatur kleine und kleinste Flüssigkeitsmengen präzise erfassen kann, so weiß man insbesondere bei kleinen Mengen nicht, wieviel der Flüssigkeit in dem Zuführschlauch (z.B. Katheter) verblieben ist und welche Menge effektiv vor Ort im Körper abgegeben wurde. Die Zuführschläuche können aufgrund von Durchmesserschwankungen und Längenunterschieden unterschiedliche Fassungsvermögen aufweisen.
c) Bei Flüssigkeitsabgabe unter Überdruck erhöht sich das Problem der präzisen Volumenmessung noch, da sich die Zuführschläuche unter Druck weiten können, womit sich das Fassungsvermögen der Zuführschläuche undefiniert erhöht.
d) Bei Benutzung einer Apparatur zur Messung des abgegebenen Flüssigkeitsvolumens wird diese mit dem Wirkstoff kontaminiert und muss vor einer weiteren Benutzung gereinigt und sterilisiert werden. D.h. eine Messapparatur zur Bestimmung der Wirkstoffabgabe muss einfach zu reinigen und zu sterilisieren sein oder als Einmalapparatur benutzt werden.

Die Internationale Patentanmeldung WO 2012/156914 A2 offenbart ein Wirkstoffabgabesystem mit Ballonkatheter, bei dem der Ballonkatheter aus einem semi-compliant oder compliant Material gebildet ist und bei dem die Poren des Ballons bei Inflationsdrücken unterhalb eines ausgewählten Schwellenwerts im Wesentlichen geschlossen bleiben und sich bei höherem Druck öffnen, um den Wirkstoff in Form von Strahlen einer Wirkstofflösung abzugeben, die das umgebende Gewebe ohne wesentliches Trauma durchdringen.

WO 2012/156914 A2 offenbart 0,8 µm bis 5 µm als Porendurchmesser, wobei technisch zum Anmeldezeitpunkt der WO 2012/156914 A2 jedoch der Bereich größer als 2,5 µm bis 5 µm nicht realisierbar war, wie später noch genauer ausgeführt wird. Der Erfinder der vorliegenden Anmeldung hat präzise Kenntnisse über die mangelnde Realisierbarkeit von Mikrobohrungen mit Porendurchmessern im Bereich von größer 2,5 µm bis 5 µm bzw. im Bereich von 3,0 µm bis 5,0 µm, da der Erfinder diese Entwicklungsarbeiten für den Anmelder der WO 2012/156914 A2 durchgeführt hat. Daher ist auch verständlich, dass in der WO 2012/156914 A2 keine Beispiele zu Katheterballonen mit Mikrobohrungen enthalten sind, welche einen Porendurchmesser im Bereich von größer 2,5 µm bis 5 µm bzw. im Bereich von 3,0 µm bis 5,0 µm aufweisen.

Aus diesem Grund besteht das Bedürfnis, eine geeignete Vorrichtung zur Wirkstoffabgabe im Körperinneren zu entwickeln, die es erlaubt, die abgegebene Wirkstoffmenge für einen Operateur während der Behandlung auf einfache Art und Weise und unmittelbar zu bestimmen.

Somit besteht die Aufgabe der vorliegenden Erfindung in der Bereitstellung einer verbesserten Vorrichtung zur dosierten Wirkstofffreisetzung im Körperinneren, die im Leitungssystem des Patientenkörpers eine lokale kontrollierte Applikation, d.h. eine präzise Dosierung eines Wirkstoffes sicherstellt. Liegt zudem am Ort der Wirkstofffreisetzung eine Gefäßläsion vor, wie z.B. eine kalzifizierte Stenose, so kann diese mit geöffnet werden.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die vorliegende Patentanmeldung löst das Problem auf einfache Weise und ist somit auf eine Vorrichtung (1) zur dosierten Freisetzung einer Wirkstoffflüssigkeit (20) über eine Wirkstoffflüssigkeitsabgabeeinheit (50) im Körperinneren gerichtet, wobei die Vorrichtung (1) umfasst:
- eine Pumpeinheit (2) mit angeschlossenem schlauchartigen Zuführsystem (40),
- mindestens eine Drossel (7) zur Begrenzung des Volumenstroms verbunden mit einem Druckaufbau im Zuführsystem,
- einen Drucksensor (3) zur Bestimmung des durch die Pumpeinheit erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das von der mindestens einen Drossel (7) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Drosseltyp (7) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen.

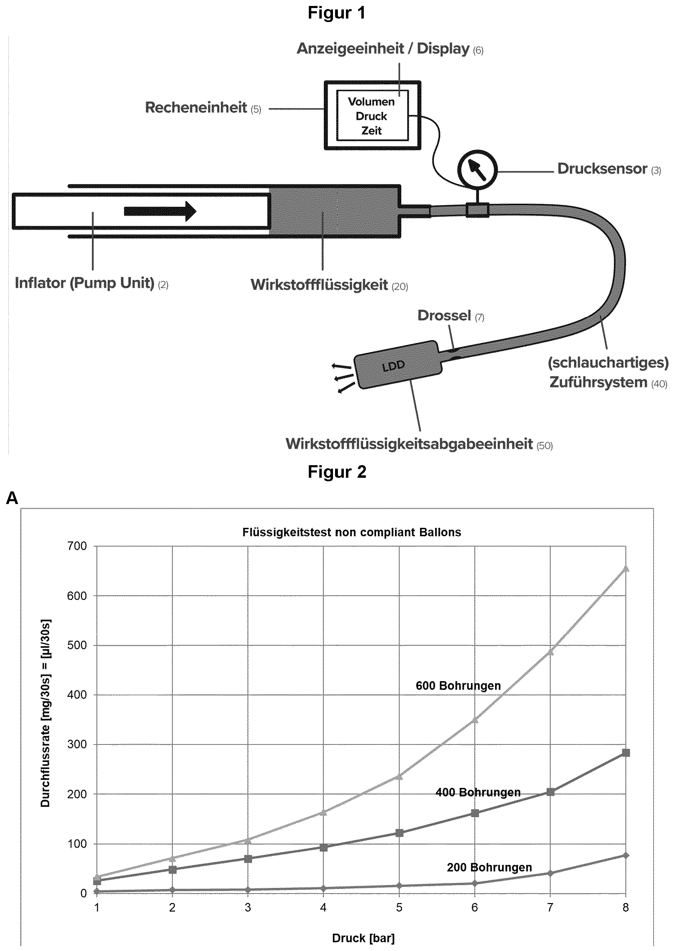

Das grundlegende Verfahrensprinzip der erfindungsgemäßen Vorrichtung (1) zur präzisen Messung und Dosierung einer Wirkstoffabgabe besteht aus einer Pumpeinheit (2), mindestens einer Drossel (7), einer Druckmesseinrichtung (3), einer Recheneinheit (5) gemäß **Figur 1**, sowie einer Anzeigeeinheit (6, Display) zur Darstellung der abgegebenen Flüssigkeitsmenge.

Der Begriff "Wirkstoffflüssigkeit" wie hierin verwendet umfasst einen oder mehrere Wirkstoffe in flüssiger Form, einen oder mehrere Wirkstoffe gelöst in einem Lösungsmittel oder Lösungsmittelgemisch wie z.B. einem Kontrastmittel, als auch einen oder mehrere Wirkstoffe suspendiert oder dispergiert in einer Flüssigkeit oder einem Flüssigkeitsgemisch. Somit beschreibt der Begriff "Wirkstoffflüssigkeit" sowohl flüssige Wirkstoffe per se, Wirkstofflösungen als auch Wirkstoffsuspensionen (d.h. lipophile Wirkstoffe in hydrophilen Lösungsmitteln oder hydrophile Wirkstoffe in lipophilen Lösungsmitteln) und Wirkstoffdispersionen (d.h. feste Wirkstoffpartikel, insbesondere Wirkstoffkristalle in einem flüssigen Medium). Hinsichtlich der Wirkstoffdispersionen unterscheidet man zwischen dispergierten Partikeln im Nanometerbereich, z.B. Partikeldurchmesser von 5 nm bis 500 nm, bevorzugt 20 nm bis 400 nm, weiter bevorzugt 50 nm bis 350 nm (d.h. Nanopartikel oder Nanospheren) und dispergierten Partikeln im Mikrometerbereich, z.B. Partikeldurchmesser von 0,5 µm bis 7,0 µm, bevorzugt 1,0 µm bis 5,0 µm, bevorzugt 1,0 µm bis 4,5 µm, bevorzugt 1,5 µm bis 4,5 µm, weiter bevorzugt 2,0 µm bis 4,5 µm (d.h. Mikropartikel oder Mikrospheren).

Der Begriff "Wirkstoffflüssigkeitsabgabeeinheit" wie hierin verwendet betrifft einen in den Körper temporär einführbaren Teil einer medizinischen Vorrichtung mit einem Drucksensor und einer Drossel zur Minderung des Volumenstroms einer unter Druck stehenden Wirkstoffflüssigkeit zwecks Abgabe eines pharmazeutischen Wirkstoffs in einer messbaren Menge. Bevorzugte Beispiele für Wirkstoffflüssigkeitsabgabeeinheiten sind Katheterballone mit Mikrobohrungen zur Abgabe einer Wirkstoffflüssigkeit, sowie mikrogebohrte Ballonkatheter mit integrierter **Lithotripsie-**Vorrichtung zur Erzeugung von Schallwellen, als auch Katheter mit als Drossel dienenden **Injektionsnadeln** zur direkten Abgabe einer Wirkstoffflüssigkeit ins Gewebe.

Über eine **Pumpeinheit,** z.B. eine Spritze und einem angeschlossenem Zuführschlauch, z.B. Katheter, der bis zur Wirkstelle im Körperinneren vorgeschoben ist, wird der Wirkstoff / das Medikament lokal abgegeben. In der Wirkstoffflüssigkeit (20) kann der Wirkstoff entweder gelöst (Lösung) vorkommen oder als feine Partikel (Dispersion) vorliegen.

Am Ende des Zuführschlauches (gegenüberliegend zur Pumpeinheit) z.B. eines Katheters, möglichst unmittelbar vor der Infusionsstelle befindet sich in einer bevorzugten Ausführungsform mindestens eine **Drossel** (7, englisch: Orifice). Die mindestens eine Drossel (7) dient dabei als Querschnittsverengung, so dass die Flüssigkeit bzw. eine nennenswerte messbare Flüssigkeitsmenge (Wirkstofflösung oder -dispersion) erst unter erhöhtem Druck durch die Drossel fließen kann.

**"drucklos"** bedeutet hier, dass die Wirkstoffflüssigkeit - nach Passieren der Drossel - ohne nennenswerten Überdruck (z.B. erzeugt durch die Pumpeinheit) an die Wirkstelle im Körperinneren abgegeben wird. Das heißt, die Flüssigkeit wird nicht durch einen zusätzlichen Druck gepumpt oder gedrückt, sondern fließt passiv zur Wirkstelle.

Der Begriff "**vernachlässigbarer kleiner Druck"** bezieht sich in diesem Zusammenhang typischerweise auf Druckbereiche, die dem Umgebungsdruck, vorzugsweise dem atmosphärischen Druck (ca. 1 bar oder 1013 hPa) nahekommen. Somit bedeutet "**vernachlässigbarer kleiner Druck"** hierin vorzugsweise ein Druck zwischen 0,08 MPa und 0,12 MPa.

Die Durchflussrate pro Zeiteinheit (z.B. µl/30 s) durch die Drossel wurde zuvor in Abhängigkeit des Flüssigkeitsdruckes, der Anzahl der Ballonbohrungen und der Viskosität der Flüssigkeit gemessen und in einer **Korrelationskurve** (30) festgehalten. Je höher der Druck und je mehr Ballonbohrungen, desto höher die Flussrate. In Figur 2A sind für eine spezifische Drossel drei Korrelationskurven für 200, 400 und 600 Ballonbohrungen bei Nutzung von Reinwasser dargestellt.

Über die Pumpeinheit wird die Wirkstoffflüssigkeit in das Zuführsystem (z.B. Katheter) gepresst. Die Flüssigkeit bzw. eine nennenswerte Flüssigkeitsmenge durchströmt die Drossel erst ab einem Schwelldruck. Der im Zuführsystem angebrachte **Drucksensor** misst permanent den Flüssigkeitsdruck und übermittelt diesen an die **Recheneinheit.** Die Recheneinheit bestimmt anhand des Flüssigkeitsdruckes und der zuvor gemessenen und gespeicherten Korrelationskurve die jeweils aktuelle Durchflussrate (z.B. µl/30 s). Mittels einer Integration der Durchflussraten über der Zeit (Addition der Durchflüsse über der Zeit) berechnet die Recheneinheit die Gesamtmenge an abgegebener Wirkstoffflüssigkeit, die in einer **Anzeigeeinheit** (Display) angezeigt wird (siehe Figur 1).

Da der Flüssigkeitsdruck im gesamten Zuführsystem von der Pumpeinheit bis zur Drossel gleich ist, wird anhand der Korrelationskurve und der Integration der Durchflussraten über der Zeit die abgegebene Wirkstoffmenge am Wirkort durch die Recheneinheit präzise ermittelt.

In einer bevorzugten Ausführungsform ist die Drossel ein querschnittsverengendes Element und kann als Kapillare oder als Ventil oder Mikrosieb oder Filter oder Mikrokanüle oder Lochblende mit einer oder mehreren Bohrungen ausgeführt sein. Andere durchflussbegrenzende Vorrichtungen sind möglich. Für die zur Anwendung kommende Ausführungsform der Drossel sind die Flussraten in Abhängigkeit der Flüssigkeitsviskosität und des Druckes zu bestimmen und in einer Korrelationskurve zu erfassen.

Hinter der Drossel besteht kein nennenswerter Druck, da die Wirkstoffflüssigkeit über einsprechende Injektions-Vorrichtungen mit einem größeren Leitungsquerschnitt (Nadel, Bohrungen im Katheter etc.) in das Gewebe oder einen Körperhohlraum abgegeben wird.

Der Vorteil dieser Apparatur aus Pumpeinheit, Zuführschlauch, Drossel, Drucksensor und Recheneinheit mit integriertem Zeitgeber ist eine einfache, kostengünstige und präzise Bestimmung der Wirkstoffabgabe am Wirkort.
- **Drosseln** sind günstig und können mit hoher Präzision hergestellt werden.
- Für einen bestimmten Drosseltyp wird die Durchflussrate in Abhängigkeit des Druckes und der Flüssigkeitsviskosität bestimmt und in einer **Korrelationskurve** festgehalten.
- **Drucksensoren** sind als kostengünstige, präzise Sensoren erhältlich.
- Die **Recheneinheit** besteht aus einem Mikroprozessor mit integriertem **Zeitgeber.** Für eine spezifische Flüssigkeit bzw. deren Viskosität wird die entsprechende Korrelationskurve der Drossel im Mikroprozessor der Recheneinheit gespeichert. Aus den gespeicherten Daten der Korrelationskurve ergibt sich jeweils die Durchflussrate (z.B. µl/30 s) für den aktuellen Druck der Pumpeinheit. Aus den Durchflussrate und den jeweiligen Zeitdauern berechnet/integriert der Mikroprozessor die insgesamt abgegebene Flüssigkeits-/Wirkstoffmenge, die in einer Anzeigeeinheit (Display) angezeigt wird.

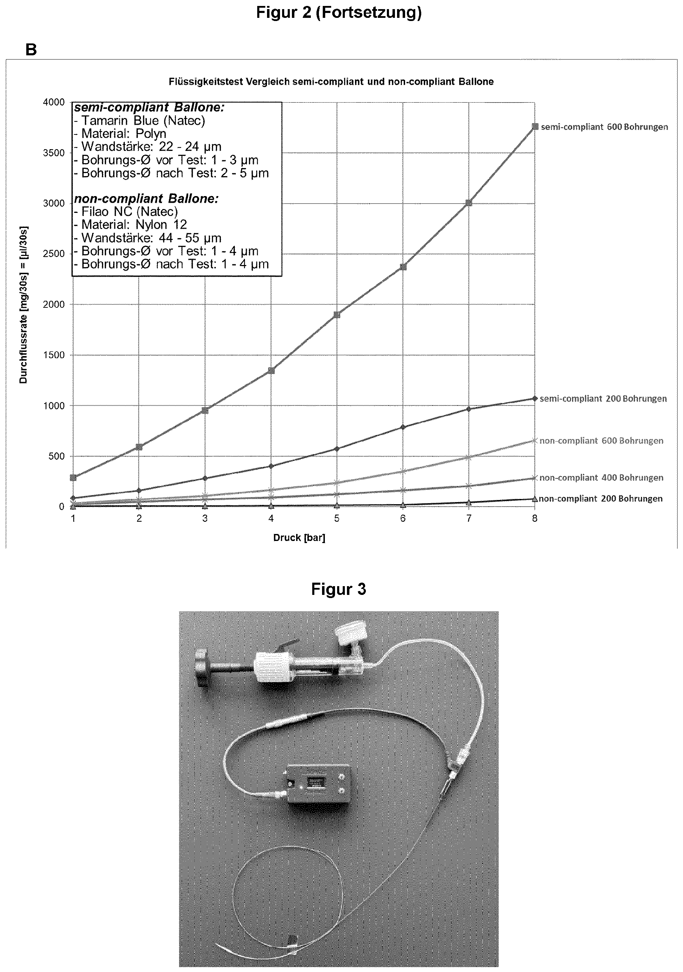

In einer Ausführungsform der erfindungsgemäßen Vorrichtung sind alle Komponenten der Vorrichtung separat voneinander getrennt (Figuren 1 und 3).

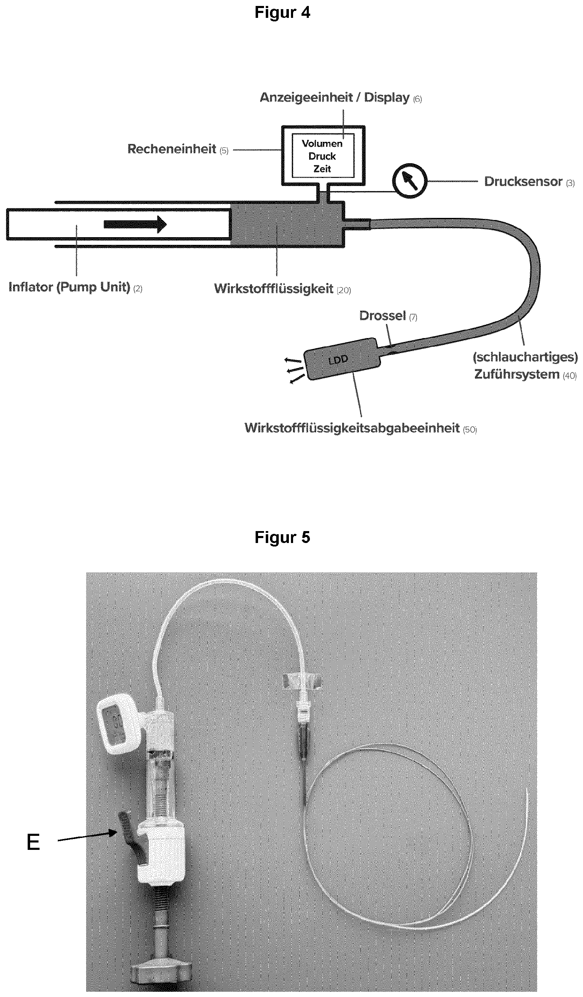

In einer bevorzugten Ausführungsform der erfindungsgemäßen Vorrichtung (1) sind die Pumpeinheit, der Drucksensor, die Recheneinheit und die Anzeigeeinheit in einer Einheit zusammengefasst (Figur 4).

Für die **Pumpeinheit** sind verschiedene Ausführungsformen möglich. So können als Pumpeinheit z.B. Kolbenspritzen dienen oder motorisch angetriebene Pumpen, die die Wirkstoffflüssigkeit aus einem Vorratsbehälter / Reservoir in den Zuführschlauch drücken.

Vorzugsweise besteht die Pumpeinheit aus einem Inflator wie er typischerweise zur Expansion von Ballonkathetern in der Kardiologie benutzt wird. Dabei ähnelt die Pumpeinheit einer übergroßen Spritze, wobei die Wirkstoffflüssigkeit über den Spritzenkolben in den Spritzenzylinder eingesogen wird (siehe Figur 5).

Der Spritzenkolben hat ein Außengewinde, d.h. ist als Gewindespindel ausgebildet und stützt sich an einem Gewindemutterelement ab, das sich am hinteren Ende des Spritzenzylinders befindet. Die Flüssigkeitsabgabe bzw. der Druckaufbau erfolgt indem der Spritzenkolben in den Spritzenzylinder hineingeschraubt wird und so die Wirkstoffflüssigkeit aus dem Spritzenzylinder herauspresst. Über das Schraubengewinde können hohe Drücke aufgebaut werden.

Durch einen Entriegelungs-/Entsperrungshebel (in Figur 5, Buchstabe E) kann die Gewindeverbindung zum Spritzenkolben jederzeit ausgerastet und somit der Druck im Spritzenzylinder aufgehoben werden.

In einer Ausführungsform befindet sich der **Drucksensor** vorzugsweise in der Pumpeinheit unmittelbar am Pumpenausgang. Alternativ kann sich der Drucksensor auch in dem Zuführschlauch/Katheter befinden und mit einem Kabel mit der Recheneinheit verbunden sein.

Vorzugsweise wird die **Recheneinheit** (5) mit **digitaler Anzeige** (6) ebenfalls in die Pumpeinheit integriert. Die Recheneinheit ist vornehmlich mit einer CPU, einem Speicher und einem **USB-Anschluss** ausgestattet, um Daten z.B. der Korrelationskurve und des Schwelldruckes in die Recheneinheit ein- bzw. auslesen zu können (siehe Figur 5).

In der digitalen **Anzeigeeinheit** (6) (Display) wird vorzugsweise u.a. die Zeitdauer, bei der sich der Druck in der Pumpeinheit oberhalb eines vorgegebenen Schwelldruckes befindet, angezeigt. Diese Dauer entspricht der Behandlungsdauer, bei der eine nennenswerte Wirkstoffabgabe erfolgt. Außerdem wird der jeweils aktuelle Flüssigkeitsdruck in Pumpeinheit und Zuführsystem angezeigt.

Das insgesamt abgegebene Flüssigkeitsvolumen wird mittels **Recheneinheit** (5), die optional in der Pumpeinheit integriert ist, den Flüssigkeitsdrücken und zugehörigen Zeitdauern und den gespeicherten Daten der Korrelationskurve (30) rechnerisch ermittelt und in der Anzeigeeinheit (Display) angezeigt. Die für die Berechnung des Flüssigkeitsvolumens benötigten Daten der Korrelationskurve sind zuvor als Datenfile in die Recheneinheit mittels Datenkabel über z.B. einem USB-Anschluss hochgeladen und im Speicher gespeichert worden.

In der **Korrelationskurve** ist die Durchflussrate pro Zeiteinheit (z.B. µl/30 s) durch die Drossel in Abhängigkeit des Flüssigkeitsdruckes und der Viskosität der Flüssigkeit festgehalten.

Eine solche Vorrichtung (1) zur präzisen Wirkstoffabgabe (Pumpeinheit/Inflator mit integriertem Drucksensor und Recheneinheit mit digitaler Anzeigeeinheit [Display]) kann von dem behandelnden Arzt auf einfache Weise eingesetzt werden. Ein großer anlagentechnischer Aufwand ist nicht notwendig. Die Apparatur ist vorzugsweise ein Einmalprodukt, da ansonsten nach erstmaliger Befüllung mit einer Wirkstoffflüssigkeit eine aufwändige Reinigung und Sterilisation vor der erneuten Benutzung erfolgen müsste.

Mittels einer **Drossel** nahe dem Wirkort der Medikamentenabgabe und der einfachen Apparatur aus manueller Pumpeinheit, Drucksensor und digitaler Anzeige von Behandlungsdauer, Druck und abgegebenem Wirkstoffvolumen können im menschlichen Körper an nahezu beliebiger Stelle, die z.B. durch Blutgefäße, Harnröhre etc. erreichbar sind, mittels eines Zuführschlauches/Zuführkatheters Wirkstoffflüssigkeiten lokal präzise kontrolliert abgegeben werden. Die Wirkstoffflüssigkeiten bestehen dabei aus Flüssigkeiten, in denen die Wirkstoffe gelöst oder dispergiert sind.

Für unterschiedliche Wirkstoffflüssigkeiten und unterschiedliche Drosseln existieren verschiedene Korrelationskurven. Die Korrelationskurven werden zuvor ermittelt, wobei für jede Flüssigkeitsviskosität und jede Drossel separate Korrelationskurven bestehen. Die Korrelationskurven sind vorzugsweise nicht als Kurven, sondern als Zahlenreihen der Durchflussrate in Abhängigkeit vom Druck ermittelt und als Datenfile in der Recheneinheit gespeichert.

Die Daten der Korrelationskurve können in der Recheneinheit für eine bestimmte Wirkstoffflüssigkeit und eine Drossel fest hinterlegt sein. Für ein flexibles System ist es aber vorteilhaft, vor der Benutzung des Wirkstoffabgabesystems die Daten der Korrelationskurve je nach verwendeter Wirkstoffflüssigkeit und Drossel in die Recheneinheit mittels einer externen Datenübertragungseinheit einzulesen und im Mikroprozessor der Recheneinheit zu speichern.

Die **externe Datenübertragungseinheit** ist vorzugsweise ein Rechner, Laptop, Tablet oder Notebook etc. mit Lesegerät (Kamera). Für die Datenübertragungseinheit existiert eine Software, die automatisch beim Anschalten der Einheit, z.B. durch den Arzt, gestartet wird.

Die Software lädt vorzugsweise per Onlineverbindung eine Datendatei mit sämtlichen Korrelationskurven und den dazugehörigen Schwelldrücken aus dem Internet herunter. Dieser Download der Korrelationskurven findet entweder manuell auf Anforderung durch den behandelnden Arzt oder automatisch beim Anschalten der Datenübertragungseinheit z.B. über W-Lan statt.

Die Datendateien sollten vorzugsweise verschlüsselt sein und nur von der Software der Datenübertragungseinheit gelesen werden können.

In gelieferten Flüssigkeitskapseln oder -behältern befindet sich die Wirkstoffflüssigkeit, die in die Pumpeinheit/Inflator eingesogen wird. Mit dem Lesegerät (Kamera) der Datenübertragungseinheit werden die QR-Codes (Barcodes) sowohl der Wirkstoffkapsel als auch der angeschlossenen Drossel gelesen. Der Barcode der Wirkstoffkapsel liefert im Wesentlichen die Viskosität der Flüssigkeit und der Barcode der angeschlossenen Drossel die Korrelationskurven in Abhängigkeit der Viskosität, d.h. die Durchflussraten in Abhängigkeit von Druck und Viskosität. Anhand beider QR-Codes wird automatisch (keine manuelle Beeinflussung) die "richtige" Korrelationskurve für die Wirkstoffflüssigkeit aus dem gespeicherten Datenbestand der Datenübertragungseinheit ausgewählt und die Daten (Wirkstoffbezeichnung, Korrelationskurve, Schwelldruck) im Monitor der Datenübertragungseinheit angezeigt.

Bei anschließender Verbindung der Datenübertragungseinheit z.B. mit dem USB-Anschluss der Recheneinheit erkennt die Software der Datenübertragungseinheit die Verbindung und überträgt (Upload) auf Bestätigung durch den Arzt die Daten der Korrelationskurve und des Schwelldruckes an die Recheneinheit. Bei korrekter Übertragung der Daten erfolgt ein Hinweis z.B. ein Signalton an der Datenübertragungseinheit. Danach kann die Pumpeinheit mit integrierter Recheneinheit zur Applikation der Wirkstoffflüssigkeit benutzt werden.

Die Pumpeinheit mit integrierter Recheneinheit wird in einer sterilen Verpackung angeliefert und kann nur einmal benutzt werden, da die Pumpeinheit nach einmaliger Benutzung mit der eingesogenen Wirkstoffflüssigkeit kontaminiert ist. Bei Anlieferung sind vorzugsweise weder Korrelationskurve noch Schwelldruck in der Recheneinheit hinterlegt, sondern werden kurz vor der Anwendung in die Recheneinheit hochgeladen. Zur Sicherheit, dass die Daten in der Recheneinheit auch tatsächlich hochgeladen sind, werden in der Anzeigeeinheit erst nach dem Daten-Upload Zeit-, Druck- und Volumenwerte angezeigt.

Über die Software der Datenübertragungseinheit sollte die Auslesung oder auch Löschung der in der Recheneinheit gespeicherten Werte möglich sein (Nullwertübertragung). Auch ein Ausdruck der benutzten Korrelationskurve und des Schwelldruckes sind für Archivierungszwecke vorzusehen.

Für den Fachmann ist leicht ersichtlich, dass die **Ausgestaltung der Drossel (7)** (Drosseltyp) vom jeweiligen Anwendungsfall im Körper abhängt. Grundsätzlich ist aber jede Art von Querschnittsverengung, die den Flüssigkeitsdurchfluss reduziert, verwendbar. Die Drosseln müssen sehr klein sein, um am Ende des Zuführschlauches eingesetzt werden zu können. Drosseln können aus allen technisch verfügbaren Werkstoffen bestehen, solange eine reproduzierbare Herstellung möglich ist.

Als "Drossel" wird hier allgemein ein örtlicher Strömungswiderstand verstanden. Im Rahmen der erfindungsgemäßen Vorrichtung sind mit "Drossel" alle Arten von Querschnittsverengungen gemeint. In einer Ausführungsform ist die Drossel eine Blende (sprungartige Querschnittsverengung), eine Düse, eine Kapillare (mit weitgehend linearer Abhängigkeit des Volumenstroms von der Druckdifferenz, sogenannte "Laminar-Drossel").

In einer weiteren Ausführungsform ist die Drossel (7) u.a. als präzise gebohrte Lochblende, Spalt, Nadelventil oder Düse ausgestaltet. Eine weitere Ausführungsform sind **Kapillare,** d.h. Röhrchen mit einem kleinen konstanten Durchmesser (sogenannte Laminar-Drossel) oder **Düsen,** d.h. Röhrchen mit einer konischen Verengung. Als Querschnittsverengung sind auch eine oder mehrere kleinste Kapillar-**Nadeln** oder Injektionsnadeln denkbar, die am Ende des Zuführungsschlauches angebracht sind, am Wirkort im Körper durch einen entsprechenden Mechanismus ausgefahren werden und die Wirkstoffflüssigkeit direkt in das Gewebe abgeben. Der Innendurchmesser aller Kapillar- bzw. Injektions-Nadeln ist so klein, dass die Nadeln insgesamt als Drossel wirken.

Vor bzw. in der Drossel baut sich bei Wirkstoffabgabe ein Druck auf, während hinter der Drossel kein oder nur ein sehr geringer Druck herrscht.

In einer Ausführungsform befindet sich (unmittelbar) hinter einer solchen Drossel (7) die **Wirkstoffflüssigkeitsabgabeeinheit** (50) zur Wirkstoffabgabe an das Gewebe oder in einen Hohlraum, die aus unterschiedlichen Komponenten bestehen kann. Ausführungsformen zur Wirkstoffabgabe sind Injektionsnadeln oder ein Ballon mit Bohrungen zur gleichmäßigen Wirkstoffabgabe über dem Umfang. Ebenso sind sterile **Vliese** oder **Filze** eine Ausführungsform der Wirkstoffabgabe. Die Wirkstoffflüssigkeit infiltriert das Vlies bzw. den Filz und diffundiert von dort in das umliegende Gewebe.

Eine insbesondere bevorzugte erfindungsgemäße Ausgestaltung der Drossel sind Mikrobohrungen in einem mit Mikrobohrungen versehenen Katheterballon, im folgenden **Drug Delivery Balloon** oder DDB genannt. Einige 10 bis einige 100 Bohrungen, d.h. 10 bis 1000 Bohrungen, bevorzugt 15 bis 900 Bohrungen, weiter bevorzugt 20 bis 800 Bohrungen, weiter bevorzugt 25 bis 700 Bohrungen, weiter bevorzugt 30 bis 600 Bohrungen, weiter bevorzugt 35 bis 500 Bohrungen, weiter bevorzugt 40 bis 400 Bohrungen sind auf dem Umfang und über die Länge des Ballons des Katheterballons verteilt, vorzugsweise gleichmäßig verteilt. Die Mikrobohrungen sind im Durchmesser so klein, dass trotz der Vielzahl der Bohrungen ein hoher Flüssigkeitsdruck im Zuführschlauch bzw. Katheterballon bei Flüssigkeitszufuhr durch die Pumpeinheit entsteht. Anhand des gemessenen Flüssigkeitsdruckes und der in der Recheneinheit gespeicherten Korrelationskurve wird die Durchflussrate aller Mikrobohrungen von der Recheneinheit ermittelt. Durch Integration der Durchflussraten über der Zeit wird das insgesamt abgegebene Flüssigkeitsvolumen von der Recheneinheit berechnet und in der Anzeigeeinheit (Display) dargestellt.

Vorzugsweise sind die Mikrobohrungen gleichmäßig über dem Ballonumfang verteilt. Jede andere Anordnung der Mikrobohrungen über der Ballonlänge und dem Ballonumfang ist möglich, um z.B. gezielt einseitig eine größere oder kleinere Wirkstoffabgabe zu bewirken.

Erfindungsgemäß sind die Durchmesser der Mikrobohrungen mit 3,0 µm bis 7,5 µm, bevorzugt 3,0 µm bis 7,0 µm, bevorzugt 3,5 µm bis 7,0 µm, bevorzugt 3,5 µm bis 6,5 µm, oder 4,0 µm bis 7,0 µm, oder 4,0 µm bis 6,5 µm, vorzugsweise 4,0 µm bis 6,0 µm sehr klein aus den folgenden Gründen:
- Mit Katheterballonen werden Gefäßverengungen im Körper geweitet, wofür im Regelfall Ballondrücke von größer 5 bar erforderlich sind. Katheterballone zur Öffnung von Stenosen in Blutgefäßen weisen typischerweise maximale Arbeitsdrücke (rated pressure) bis 16 bar auf, spezielle Hochdruckballone bis über 30 bar. Bei diesen Drücken führen Bohrungen gleich oder größer 8 µm:
   a) zu einem Flüssigkeitsstrahl (Jetstream) aus jeder einzelnen Bohrung, der die Gefäß- bzw. Gewebewandung beschädigen kann,
   b) zu hohen Flussraten pro Bohrung und damit zu einer Beschränkung auf wenige Bohrungen in dem Ballon des Katheterballons, da die maximale Flüssigkeitszufuhr über den dünnen Zuführschlauch begrenzt ist. Eine gleichmäßige Wirkstoffverteilung über einen größeren Behandlungsbereich kann mit wenigen Bohrungen, d.h. vor allem bei weniger als 10 Bohrungen nicht gewährleistet werden.
- Mit Katheterballonen werden vorzugsweise Blutgefäße behandelt. Schon bei einem geringen Flüssigkeitsdruck legt sich die Ballonwandung an die Gefäßwandung. Gesunde Gefäßwandungen sind sehr glatt, im Durchmesser in Grenzen flexibel und dichten jeglichen Blutfluss zwischen Ballon und Gefäß ab. Strömt Flüssigkeit aus den Mikrobohrungen des Ballons in Richtung Gefäßwandung, so kann die Flüssigkeit nicht abfließen; es bilden sich direkt oberhalb der Mikrobohrungen kleine Flüssigkeitskammern, die zu Gefäßausstülpungen führen. Ab einer gewissen Größe führen diese Ausstülpungen zu Dissektionen der empfindlichen Gefäßwandung. Aus diesem zusätzlichen Grund sollten die Bohrungen sehr klein, kleiner als 8,0 µm, vorzugsweise kleiner als 7,6 µm, bevorzugt nicht größer als 7,5 µm sein, um die örtliche Flüssigkeitsabgabe aus einer Mikrobohrung zu begrenzen.
- Ferner haben sich Bohrungsdurchmessern von < 3,0 µm oder von < 2,8 µm als ungeeignet herausgestellt. Es tritt so gut wie keine Flüssigkeit mehr durch die Bohrungen, der Druckverlust ist zu hoch. Zur Kompensation des hohen Druckverlustes an den Bohrungen müsste mit sehr, sehr hohen Systemdrücken gearbeitet werden, was die Zuführleitungen überlasten würde.
- In der Regel ist der Wirkstoff nicht in der abgegebenen Flüssigkeit gelöst. Eine solche Wirkstoffabgabe hätte nur eine kurzzeitige Wirkung. Um eine Wirkung über einen längeren Zeitraum im Gewebe zu erzeugen, sind die Wirkstoffe häufig als schwerlösliche **Mikro- bzw. Nanopartikel** (sogenannte Sphere) der (Träger-) Flüssigkeit zugegeben (Dispersion). Die Schwerlöslichkeit wird durch Kristallisierung der Wirkstoffe erreicht und/oder durch Einkapselung in schwerlöslichen Medien. So werden z.B. als zytostatisches Medikament Sphere aus Sirolimuskristallen verwandt (Firma Cordis/Medalliance, Firma Concept Medical). Aktuell existieren zwei Typen von Sphere mit unterschiedlichen Wirkmechanismen:
   a) Nanosphere haben einen Durchmesser kleiner 0,3 µm (Firma Concept Medical, Drug Coated Ballon "Magic Touch"). Nanosphere kleiner/gleich 0,3 µm (≤ 0,3 µm) sind gewebegängig, d.h. sie diffundieren in das Gewebe und lösen sich dort langsam auf. Nanosphere neigen zur Clusterbildung, weshalb die Bohrungsdurchmesser um den Faktor 10 größer sein müssen, um ein Verstopfen der Mikrobohrungen zu vermeiden, mithin müssen die Bohrungen mindestens 3 µm groß sein.
   b) Mikrosphere in der Größe von wenigen Mikrometern sind mit einem zellanhaftenden Träger versehen, um an der Gewebewandung zu haften (Cordis/Medalliance, Drug Eluting Ballon "Selution"). Entscheidend ist die Kristallinität des Wirkstoffes, um sich langsam aufzulösen, wodurch die Langzeitwirkung entsteht. Für diese Mikrosphere müssen die Mikrobohrungen mindestens 3,0 µm, verzugsweise größer als 3,5 µm oder 4,0 µm, weiter bevorzugt größer als 4,5 µm oder 5,0 µm oder 5,5 µm groß sein.

Nur der in dieser Patentanmeldung genannte Bohrungsdurchmesserbereich von 3,0 µm bis 7,5 µm ist sowohl für Nanosphere als auch Mikrosphere geeignet. Nanosphere, die sich vorzugsweise clustern, verstopfen Bohrungen < 3 µm und Mikrosphere mit Durchmessern von ≥ 4 µm können in Bohrungen ≤ 5 µm leicht hängen bleiben. Von dem in Patentanmeldung WO 2012/156914 A2 genannten Bohrungsdurchmessern von 0,8 - 5 µm ist somit nur der Bereich von 3 - 5 µm für den Einsatz von Sphere geeignet. Dieser Durchmesserbereich ließ sich aber zum Zeitpunkt der Patentanmeldung nicht ausführen (siehe Beispiel 3).

Zur Sicherstellung, dass die über die Mikrobohrungen zugeführte Flüssigkeit sich gut verteilen oder auch überschüssige Flüssigkeit abfließen kann, sind folgende Ausführungsformen der mikrogebohrten wirkstofffreisetzenden Ballone (Drug Delivery Ballone) vorgesehen:
- Die Ballone werden mit einem dünnen **Vlies** oder **Filz** oder jeder anderen offenporigen Schicht überzogen, so dass die aus den Mikrobohrungen austretende Flüssigkeit sich an der Gewebewandung verteilen und auch abfließen kann.
- Die Ballone werden mit einem **Gewebe** oder einen **Metallnetz** überzogen (wie in WO 2014/167050 A1 beschrieben: mit Mikrobohrungen und einem Metallnetz versehener Katheterballon), so dass die aus den Mikrobohrungen austretende Flüssigkeit sich an der Gewebewandung verteilen und abfließen kann.

Durch die erfindungsgemäße Vorrichtung wird die lokale Abgabe einer Wirkstoffflüssigkeit und damit auch die Medikamentendosierung eines wirkstofffreisetzenden Ballons (Drug Delivery Balloons) präzise erfasst, womit die Behandlung des Patienten entscheidend verbessert wird, indem Über- und Unterdosierungen vermieden werden. Darüber hinaus wird die Prozedur der Gefäßbehandlung vereinfacht.

Die vorliegende Erfindung betrifft daher eine Vorrichtung (1) bzw. konkreter eine Inflationsvorrichtung (1) für Mikrobohrungen (11) aufweisende non-compliant Katheterballone (10) zur dosierten Freisetzung einer Wirkstoffflüssigkeit (20) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen non-compliant Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen.

Die Vorteile, die die erfindungsgemäße Inflationsvorrichtung (1) für den Einsatz von DDBs (Drug Delivery Ballons) in der Behandlung von Organ- oder Gewebe-/Gefäßerkrankungen bringt, sind vielfältig:
a) Die an das Organ bzw. die Gefäßwandung abgegebene Medikamentendosis wird dank der erfindungsgemäßen Vorrichtung präzise ermittelt und angezeigt. Das Medikament wird ausschließlich an der erkrankten Gewebe-/Gefäßstelle erst bei Inflation des gebohrten Ballons abgegeben. Beim Vorschieben des Katheterballons von der perkutanen Gefäßpunktion bis zur Läsion wird kein Medikament abgegeben.
b) Die Wirkstoffabgabe wird individuell während der Behandlung der Läsion vom behandelnden Arzt mittels Inflator (2) über den Inflationsdruck und die Inflationsdauer gesteuert. Sie wird von der erfindungsgemäßen Vorrichtung während der Gefäßbehandlung durch die Recheneinheit (5) ermittelt und in der Anzeigeeinheit (6) angezeigt.
c) Nur bei an der Gefäßwandung anliegendem Ballon und Druckerhöhung wird Medikament durch die Mikrobohrungen abgegeben. Bei einer zwischenzeitlichen Deflation des Katheterballons wird kein Medikament abgegeben.
d) Die Behandlung einer Läsion, d.h. die Abgabe des Medikaments an die erkrankte Gewebewandung, kann mehrfach unterbrochen werden, um durch eine kurzzeitige Deflation des Ballons einen Durchfluss zu ermöglichen und damit eine Unterversorgung der nachgelagerten Gewebe zu vermeiden. So kann z.B. die Aufdehnung einer Stenose in einem Herzkranzgefäß mehrfach unterbrochen werden, um das Risiko eines Herzinfarktes durch eine längere Gefäßblockade zu vermeiden. Der Katheterballon kann also mehrfach kurzzeitig inflatiert werden, anstelle einer einmaligen langen Inflation bei den derzeit zum Einsatz kommenden Drug Coated Ballonen (DCBs). Vom behandelnden Arzt wird die Medikamentenabgabe an die Gefäßwandung durch die mehrfache Inflation des gebohrten Ballons über die gesamte Behandlungsdauer gesteuert und von der erfindungsgemäßen Inflationsvorrichtung (1) insgesamt erfasst und angezeigt.
e) Mit einem einzigen mikrogebohrten Katheterballon können auch eine längere Läsion oder mehrere Läsionen eines Patienten nacheinander behandelt werden.
f) Das Medikament wird sehr gleichmäßig über Umfang und Länge des Ballons an die Gefäßwandung abgegeben, da eine Vielzahl (10 bis 1000, bevorzugt 15 bis 900, weiter bevorzugt 20 bis 800, weiter bevorzugt 25 bis 700, weiter bevorzugt 30 bis 600, weiter bevorzugt 35 bis 500, weiter bevorzugt 40 bis 400) Mikrobohrungen gleichmäßig auf dem Ballon verteilt sind.
g) Das an die Gefäßwandung abzugebende Medikament kann frei gewählt werden. Der Ballonkatheter wird erst unmittelbar vor dem Eingriff mit einer gebrauchsfertigen Wirkstoffflüssigkeit, die sich vorzugsweise in einer Kapsel oder Behälter befindet, beladen.
h) Die Haltbarkeitsdauer (shelf life) des Katheterballons ist nicht durch das Medikament beschränkt, da der Katheterballon und das Medikament in der Wirkstoffkapsel bis kurz vor der Anwendung separat gelagert werden.

In einer bevorzugten Ausführungsform wird ein mikrogebohrter, **non-compliant Katheterballon,** dessen Mikrobohrungen ihren Durchmesser auch bei hohen Drücken von z.B. 20 bar (2 MPa) beibehalten, mit der erfindungsgemäßen Inflationsvorrichtung (1) verwendet. Die erfindungsgemäßen mikrogebohrten, non-compliant Katheterballone sind zur Applikation einer Wirkstoffflüssigkeit (20) bei einem Balloninnendruck von 2 bar (0,2 MPa) bis 30 bar (3 MPa) geeignet.

Im Gegensatz zu semi-compliant Ballone, die um 20-30 % überdehnen, neigen non-compliant Ballone nur zu einer Erweiterung bei Druckaufbau um weniger als 20%, d.h. von 3% bis < 20% oder 4% - 19% oder 5% - 19 % oder 5% - 18% oder 5% - 17% oder 5% - 16% oder 5% - 15%. Diese geringe Überdehnung ermöglicht eine präzise Kontrolle der Ballonabmessungen.

Die Erfinder konnten zudem zeigen, dass sich die Bohrungen in non-compliant Ballonen bei der Entfaltung unter Dilatationsdrücken nicht irreversibel vergrößern, siehe Legende in Figur 2B. Der ursprüngliche Bohrungsdurchmesser bleibt bei non-compliant Ballonen auch nach wiederholter Dilatation konstant. Das sorgt für eine gleichbleibende und vorhersagbare Durchflussrate von non-compliant Ballonen. Nach dreifacher Dilatation und selbst nach fünffacher Dilatation betrug die irreversible Weitung der Mikrobohrungen weniger als 5% des Bohrungsdurchmessers, bevorzugt weniger als 4% des Bohrungsdurchmessers, weiter bevorzugt weniger als 3% des Bohrungsdurchmessers, weiter bevorzugt weniger als 2,5% des Bohrungsdurchmessers, weiter bevorzugt weniger als 2% des Bohrungsdurchmessers.

Für mikrogebohrte **semi-compliant Ballone** zeigt Figur 2B dagegen eine deutlich höhere Durchflussrate im Vergleich mit non-compliant Ballonen trotz gleichem Bohrungsdurchmesser und gleicher Bohrungsanzahl. Mit steigendem Inflationsdruck weiten sich die Bohrungen zunehmend, womit die Durchflussrate exponentiell ansteigt und dann nur noch unzureichend präzise gemessen werden kann.

**Überraschenderweise** bleiben bei hohen Drücken - Dilatationsdrücke koronarer Katheterballone liegen üblicher Weise bei ca. 16 bar (1,6 MPa) - einmal geweitete, d.h. expandierte **semi-compliant Ballone** und die bei Dilatation aufgeweiteten Mikrobohrungen auch nach der Deflation weiterhin in Grenzen geweitet. Beispielsweise beträgt der gemessene Bohrungsdurchmesser vor der Durchflussmessung 1-3 µm. Nach einer Inflation mit bis zu 8 bar betragen die Bohrungsdurchmesser im drucklosen Zustand 2-5 µm, siehe Figur 2B. Bei einer erneuten Durchflussmessung ist der Durchfluss dann entsprechend höher. Für semi-compliant Ballone ist bei mehrfacher Dilatation die genaue Bestimmung der Durchflussrate anhand des Druckes mittels der erfindungsgemäßen Inflationsvorrichtung deutlich erschwert bzw. wird mit jeder weiteren Dilatation ungenauer. Gerade die Wirkstoffabgabe durch mehrfache Ballondilatation ist aber ein wesentlicher Vorteil der gebohrten non-compliant Katheterballone gegenüber den semi-compliant Katheterballonen als auch gegenüber den derzeit eingesetzten wirkstoffbeschichteten Katheterballonen, den sogenannten Drug Coated Ballonen (DCB), die nur einmal dilatiert werden können, um die aufgebrachte Medikamentendosis an die Gefäßwandung abzugeben.

Durch die stabile Dimension und den konstanten Durchfluss der non-compliant Ballone können Wirkstoffe genauer dosiert und gezielt abgegeben werden, ohne dass ungewollte Änderungen der Durchflussraten, wie sie bei semi-compliant Ballonen auftreten können, zu berücksichtigen sind. Bei einem erneuten Aufdehnen von semi-compliant Ballonen werden die zuvor gemessenen Durchflussraten bereits bei geringeren Flüssigkeitsdrücken erreicht. Eine präzise mehrfache Wirkstoffabgabe mittels semi-compliant Ballonen erfordert die aufwendige Einbeziehung der irreversiblen Bohrungsweitung bei der Druckerhöhung, was häufig im Stand der Technik vernachlässigt wird, wie etwa in WO 2012/156914 A2. Bei wiederholten Ballondilatationen muss daher die irreversible Weitung der Bohrungsdurchmesser mit berücksichtigt werden.

Diese Eigenschaften machen non-compliant Katheterballone besonders für eine kontrollierte und wiederholbare Anwendung mit der erfindungsgemäßen Inflationsvorrichtung (1) geeignet.

Die vorliegende Erfindung betrifft daher eine Vorrichtung (1), bzw. präziser eine Inflationsvorrichtung (1) für Mikrobohrungen (11) aufweisende non-compliant Katheterballone (10) zur mehrfachen dosierten Freisetzung einer Wirkstoffflüssigkeit (20) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen.

Bei der Wirkstoffflüssigkeit handelt es sich vorzugsweise um eine Lösung eines **zytotoxischen oder zytostatischen Antirestenosewirkstoffs** wie beispielsweise Rapamycin, Methylrapamycin, Biolimus A9, Deforolimus, Everolimus, Myolimus, Novolimus, Pimecrolimus, Ridaforolimus, Tacrolimus, Temsirolimus, Zotarolimus, Paclitaxel, Taxotere, Baccatin, 7-xylosyl-10-Deacetyltaxol, Cephalomannin, 10-Deacetyl-7-epitaxol, 7-Epitaxol, 10-Deacetylcephalomannin, 7-Desoxy-docetaxol, 7,8-Zyklopropataxane, Fluortaxole, 9-Deoxotaxan oder **anti-inflammatorische/entzündungshemmende Mittel** wie Dexamethason, Hexestrol, Methimazol, Betamethason, Triamcinolon, Fluocinonid, Prednisolon, Methylprednisolon, Hydrocortison, Fluorometholon, Beclomethasondipropionat, Estriol, Clobetasol, Diflorasondiacetat, Halbetosalpropionat, Amicinonid, Desoximetason, Halcinonid, Mometasonfuroat, Fluticasonpropionat, Flurandrenolid, Clocortalon, Predincarbat, Aclometasondipropionat, Desonid oder **Thrombolytika** wie Heparin, Alteplase, Tenecteplase und Streptokinase oder **gentherapeutische Mittel** oder **Antitumor Medikamente** wie beispielsweise Actinomycin D, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, Docetaxel, Doxorubicin (Adriamycin), Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Tioguanin, Topoisomerase-Inhibitoren, Topotecan, Treosulfan, Tretinoin, Triptorelin, Trofosfamide, Vinblastin, Vincristin, Vindesin, Vinorelbin, zytostatisch wirksame Antibiotika in einem Dispergiermittel oder Lösungsmittel wie beispielsweise einem Kontrastmittel, welches z.B. bei der Computertomographie (CT), Kernspintomographie, Magnetresonanztomographie (MRT) oder Ultraschall eingesetzt wird. Beispiele für geeignete Kontrastmittel sind Amidotrizoesäure, lotrolan, lopamidol, lodoxaminsäure, Gadopentetsäure (GaDPTA), Gadodiamid, Meglumin-Gadoterat sowie Gadoteridol als auch deren pharmazeutische Formulierungen wie z.B. lopromid.

Weiterhin haben die Erfinder **überraschenderweise** festgestellt, dass die optimalen Bohrungsdurchmesser der mikrogebohrten Ballone zwischen 3,0 µm und 7,5 µm, bevorzugt 3,0 µm bis 7,0 µm, bevorzugt zwischen 3,5 µm - 6,5 µm, weiter bevorzugt zwischen 4,0 µm und 6,0 µm, für die Verwendung mit der erfindungsgemäßen Inflationsvorrichtung (1) zur dosierten Wirkstoffabgabe liegen. Bei diesen Bohrungsdurchmessern wird bei den üblichen Katheterballondrücken von bis zu 16 bar bzw. 18 bar und den Viskositäten der Kontrastmittel ausreichend Wirkstoffflüssigkeit abgegeben. Im Stand der Technik (z.B. WO 2012/156914 A2) wurden Wirkstoffabgabesysteme getestet, die einen mikrogebohrten Katheterballon mit Mikrobohrungen mit einem Durchmesser ≤ 2,5 µm verwenden. Katheterballons mit Mikrobohrungen mit einem Durchmesser zwischen 3,0 µm und 7,5 µm oder zwischen 3,0 µm und 7,4 oder zwischen 3,0 µm und 7,0 konnten zu diesem Zeitpunkt technisch nicht mit der für die dosierte Wirkstofffreisetzung erforderliche Reproduzierbarkeit und Präzision zuverlässig hergestellt werden, wie im Folgenden noch genauer ausgeführt wird.

Bohrungsdurchmesser kleiner als 3,0 µm oder kleiner als 2,8 µm haben sich als ungeeignet herausgestellt, denn es zeigte sich, dass die Wirkstoffflüssigkeiten und alle eingesetzten Apparaturen extreme Reinheiten aufweisen müssen, um ein Verstopfen der Bohrungen zu vermeiden. Diese Reinheiten können im klinischen Alltag im Krankenhaus nicht sichergestellt werden. Außerdem muss mit hohen Flüssigkeitsdrücken gearbeitet werden, damit überhaupt genügend Flüssigkeit aus Mikrobohrungen <3,0 µm oder <2,8 µm austritt. Dennoch ist trotz hoher Flüssigkeitsdrücke von bis zu 20 bar die gesamte Durchflussrate eines solchen Katheterballons mit derart kleinen Bohrungen zu gering.

Bohrungsdurchmesser von über 7,5 µm oder über 7,7 µm haben sich aus mehreren Gründen ebenfalls als ungeeignet herausgestellt. Durch die einzelne Ballonbohrung wird zu viel Flüssigkeit abgegeben. Bohrungen > 8,0 µm werden daher im Stand der Technik (z.B. in WO 2012/156914 A2) auch häufig folgerichtig als "makroskopische" Bohrungen bezeichnet.

Besonders nachteilig von Bohrungsdurchmessern größer 7,5 µm oder größer 7,7 µm ist, dass sich keine hohen Drücke im Katheterballon aufbauen lassen, da der Zufluss zum Katheterballon durch die kleinen Leitungsquerschnitte der Katheter begrenzt ist.

Zudem kann keine gleichmäßige Wirkstoffabgabe im Gefäß, über die Vielzahl von Bohrungen größer als 7,5 µm oder 7,7 µm, die gleichmäßig auf dem Katheterballon verteilt sind, sichergestellt werden, da der gesamte Volumenstrom aller Bohrungen zu groß. Der kleine Innendurchmesser des ca. 1,5 m langen Katheters lässt hohe Volumenströme nicht zu.

Zudem sind Bohrungen größer als 7,5 µm oder 7,7 µm zu groß für die Bestimmung des Flüssigkeitsvolumenstromes über den Druck im Zuführsystem. Um einen ausreichend hohen messbaren Druckaufbau im Zuführsystem zu erzielen, muss die Querschnittsverengung, d.h. die Summe aller Bohrungen sehr klein sein. Alternativ müsste der Volumenstrom der Wirkstoffflüssigkeit sehr groß sein, was (wie zuvor beschrieben) nicht gewünscht ist.

Ferner kann bei hohen Flüssigkeitsdrücken ein Flüssigkeitsstrahl aus den Bohrungen austreten ("Jetting"), wenn sie größer als 7,5 µm oder 7,7 µm im Durchmesser sind. Der Flüssigkeitsstrahl kann zur Schädigung der Gefäßwandung führen und muss daher vermieden werden.

Darüber hinaus konnten die Erfinder in Untersuchungen weitere überraschende Ergebnisse zum optimalen Durchmesser der Bohrungen erzielen.

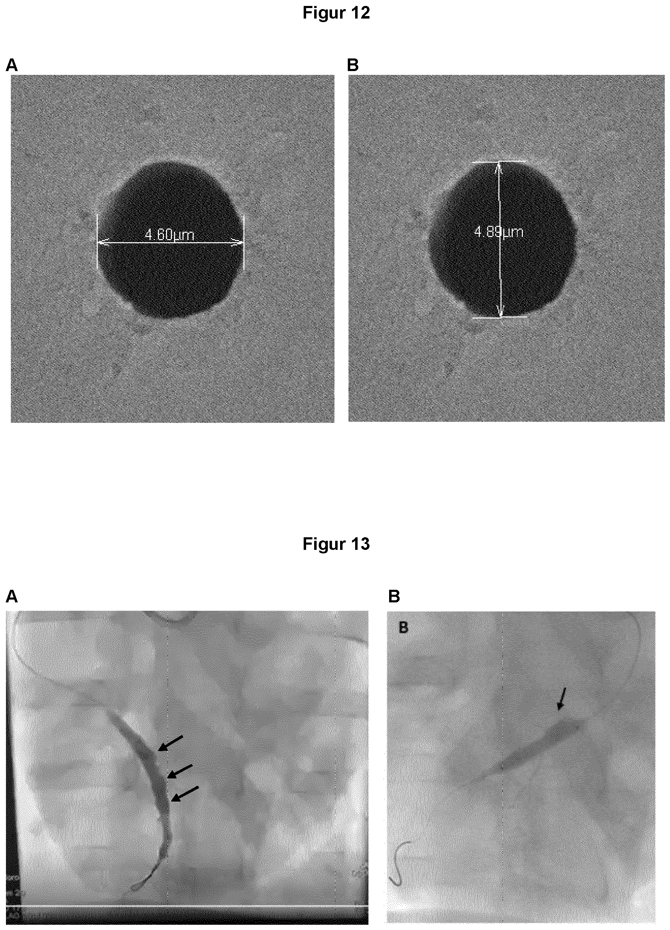

Liegt die Ballonwandung aufgrund des Balloninnendruckes an der Gefäßwandung an, können bei Bohrungsdurchmessern von ≥ 8,0 µm die hohen, lokal aus einer Bohrung abgegebenen Flüssigkeitsmengen nicht bzw. nicht schnell genug seitlich abfließen. Es kommt zu einer lokalen Ausstülpung (Vorwölbung) der Gefäßwandung mit dem Risiko von Gefäßdissektionen. Auf diese Weise lassen sich bei Tiermodellen bzw. präklinischen Untersuchungen gezielt Dissektionen erzeugen. Figur 13 zeigt derartige Dissektionen von Koronargefäßen. Deutlich sind die Gefäßausstülpungen in beiden angiographischen Bildern zu erkennen. Die hohe Durchflussraten aufgrund von zu großen Ballonbohrungen, d.h. Bohrungsdurchmesser größer 8,0 µm sind daher unbedingt zu vermeiden oder es sind entsprechende Vorkehrungen für einen Abfluss der abgegebenen Flüssigkeiten vorzusehen. Ballone mit erhöhten Flüssigkeitsabgabe aufgrund von sehr vielen Bohrungen oder großen Bohrungen sind deshalb mit einem Vlies oder einem Filz oder einem Gewebe oder einem Metallnetz (15) zu überziehen, um den Abfluss der Wirkstoffflüssigkeit zu gewährleisten.

Aufgrund der zuvor beschriebenen Untersuchungen zur erfindungsgemäßen Inflationsvorrichtung (1) liegen die optimalen Bohrungsdurchmesser daher bei Katheterballonen im Bereich von 3,0 µm bis 7,5 µm, bevorzugt 3,0 µm bis 7,0 µm, bevorzugt von 3,5 µm bis 7,0 µm, bevorzugt von 3,5 µm bis 6,5 µm, bevorzugt von 4,0 µm bis 7,0 µm, bevorzugt von 4,0 µm bis 6,5 µm, weiter bevorzugt von 4,0 µm bis 6,0 µm.

Das Merkmal >> Bohrungsdurchmesser im Bereich von X,0 µm bis Y,0 µm << wie z.B. >> Bohrungsdurchmesser im Bereich von 3,0 µm bis 7,0 µm << ist nicht so zu verstehen, dass sich in einem Katheterballon Mikrobohrungen beliebiger Durchmesser im Bereich von 3,0 µm bis 7,0 µm befinden, d.h. ein Katheterballon weist keine Mischung von Mikrobohrungen unterschiedlicher Durchmesser im Bereich von 3,0 µm bis 7,0 µm auf, also keine Mikrobohrungen von 3,0 µm und gleichzeitig von 4,0 µm und 5,0 µm und 6,0 µm und 7,0 µm. Dieses Merkmal ist so zu verstehen, dass ein Katheterballon nur Mikrobohrungen eines definierten Durchmessers im Bereich von 3,0 µm bis 7,0 µm aufweist, wie z.B. nur Mikrobohrungen mit 3,0 µm Durchmesser oder beispielsweise nur Mikrobohrungen mit 4,4 µm Durchmesser oder nur Mikrobohrungen mit 5,2 µm Durchmesser oder nur Mikrobohrungen mit 6,1 µm Durchmesser oder nur Mikrobohrungen mit 7,0 µm Durchmesser. Diese Mikrobohrungen eines definierten Durchmessers weisen natürlich eine gewisse Bohrungsgenauigkeit auf, welche erfindungsgemäß sehr gering ist und bei ±0,2 µm liegt. Somit bezeichnet das Merkmal >> Mikrobohrungen (11) mit einem Bohrungsdurchmesser im Bereich von 3,5 µm bis 6,5 µm << beispielsweise einen Katheterballon nur mit Mikrobohrungen von 5,2 µm ± 0,2 µm Bohrungsgenauigkeit <<.

Zudem liegt die bevorzugte Anzahl an Bohrungen pro Katheterballon bei 10 bis 1000, bevorzugt 15 bis 900, weiter bevorzugt 20 bis 800, weiter bevorzugt 25 bis 700, weiter bevorzugt 30 bis 600, weiter bevorzugt 35 bis 500, weiter bevorzugt 40 bis 400. Typischerweise beträgt die Anzahl der Mikrobohrungen auf der Ballonoberfläche 0,1 bis 5 Mikrobohrungen pro mm², bevorzugt 0,2 bis 4,5 Mikrobohrungen pro mm², bevorzugt 0,3 bis 4,0 Mikrobohrungen pro mm² oder 0,5 bis 4,0 Mikrobohrungen pro mm², bevorzugt 1,0 - 3,0 Mikrobohrungen pro mm² oder bevorzugt 1,5 bis 2,5 Mikrobohrungen pro mm².

In einer bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer Wirkstoffflüssigkeit (20) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen (11) mit einem Bohrungsdurchmesser im Bereich von 3,0 µm bis 7,5 µm, bevorzugt 3,0 µm bis 7,0 µm, bevorzugt von 3,5 µm bis 6,5 µm, weiter bevorzugt von 4,0 µm bis 6,0 µm aufweist.

In einer weiteren bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion** (21) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion (21) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion (21) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion (21) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion (21) anzuzeigen,
wobei der Katheterballon (10) Mikrobohrungen (11) mit einem Bohrungsdurchmesser im Bereich von 4,0 µm bis 7,5 µm, bevorzugt von 4,0 µm bis 7,0 µm, bevorzugt von 4,5 µm bis 7,5 µm, bevorzugt von 4,5 µm bis 7,0 µm, weiter bevorzugt von 5,0 µm bis 7,5 µm, bevorzugt von 5,0 µm bis 7,0 µm, weiter bevorzugt von 5,5 µm bis 7,5 µm aufweist.

In einer anderen bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Mikrospheren** (22) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Mikrospheren (22) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Mikrospheren (22) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Mikrospheren (22) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Mikrospheren (22) anzuzeigen,
wobei der Katheterballon (10) Mikrobohrungen (11) mit einem Bohrungsdurchmesser im Bereich von 3,5 µm bis 7,5 µm oder von 4,0 µm bis 7,5 µm, oder von 4,5 µm bis 7,5 µm, oder von 5,0 µm bis 7,5 µm, oder von 5,5 µm bis 7,5 µm aufweist.

Die Mikrospheren (22) besitzen Durchmesser im Bereich weniger µm. Damit die Mikrospheren (22) die Mikrobohrungen (11) ungehindert passieren können, sollten die Mikrobohrungen (11) einen um mindestens 0,5 µm oder mindestens 0,6 µm oder mindestens 0,7 µm oder mindestens 0,8 µm größeren Durchmesser als die Mikrospheren (22) haben. Dies bedeutet, dass bei Mikrospheren (22) eines Durchmessers von 3,0 µm die Mikrobohrungen (11) mindestens einen Durchmesser von 3,5 µm, bevorzugt 3,6 µm oder 3,7 µm oder 3,8 µm aufweisen sollten, d.h. in diesem Fall sollten die Durchmesser der Mikrobohrungen (11) im Bereich von 3,5 µm bis 7,5 µm liegen bevorzugt im Bereich von 3,6 µm oder 3,7 µm oder 3,8 µm bis 7,5 µm. Bei Mikrospheren (22) eines Durchmessers von 5,0 µm sollten die Mikrobohrungen (11) mindestens einen Durchmesser von 5,5 µm, bevorzugt mindestens von 5,6 µm oder 5,7 µm oder 5,8 µm besitzen, d.h. Durchmesser im Bereich von 5,5 µm bis 7,5 µm, bevorzugt von 5,6 µm bis 7,5 µm oder 5,7 µm bis 7,5 µm oder 5,8 µm bis 7,5 µm aufweisen.

Daher betrifft die vorliegende Erfindung auch eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Mikrospheren** (22) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Mikrospheren (22) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Mikrospheren (22) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Mikrospheren (22) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Mikrospheren (22) anzuzeigen,
wobei der Katheterballon (10) Mikrobohrungen (11) mit einem Bohrungsdurchmesser im Bereich von 3,5 µm bis 7,5 µm oder von 4,0 µm bis 7,5 µm, oder von 4,5 µm bis 7,5 µm, oder von 5,0 µm bis 7,5 µm, oder von 5,5 µm bis 7,5 µm aufweist und die Mikrobohrungen (11) einen um mindestens 0,5 µm oder mindestens 0,6 µm oder mindestens 0,7 µm oder mindestens 0,8 µm größeren Durchmesser als die Mikrospheren (22) haben.

In einer anderen bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Nanospheren** (23) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Nanospheren (23) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Nanospheren (23) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Nanospheren (23) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Nanospheren (23) anzuzeigen,
wobei der Katheterballon (10) Mikrobohrungen (11) mit einem Bohrungsdurchmesser im Bereich von 3,0 µm bis 7,5 µm, bevorzugt 3,0 µm bis 7,0 µm, bevorzugt von 3,5 µm bis 6,5 µm, weiter bevorzugt von 4,0 µm bis 6,0 µm aufweist.

Vorzugsweise umfasst die erfindungsgemäße Vorrichtung (1) bzw. Inflationsvorrichtung (1) für Mikrobohrungen (11) aufweisende non-compliant Katheterballone (10) zur dosierten Freisetzung einer **Wirkstoffflüssigkeit** (20)
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen mit einem Bohrungsdurchmesser im Bereich von 3,0 µm bis 7,5 µm, bevorzugt 3,0 µm bis 7,0 µm, bevorzugt von 3,5 µm bis 6,5 µm, weiter bevorzugt von 4,0 µm bis 6,0 µm aufweist und wobei nach dreifacher Dilatation oder nach fünffacher Dilatation die irreversible Weitung der Mikrobohrungen weniger als 5% des Bohrungsdurchmessers, bevorzugt weniger als 4% des Bohrungsdurchmessers, weiter bevorzugt weniger als 3% des Bohrungsdurchmessers, weiter bevorzugt weniger als 2,5% des Bohrungsdurchmessers, weiter bevorzugt weniger als 2% des Bohrungsdurchmessers beträgt.

Eine ältere Vorrichtung der ersten Generation zur Messung der Wirkstoffabgabe bei Drug Delivery Ballonen wird in den Patentanmeldungen WO 2012/156914 A2 und US 2011/160575 A1 beschrieben. WO 2012/156914 A2 offenbart die erfindungsgemäße Verwendung von semi-compliant Katheterballonen (Anspruch 1) und offenbart compliant Katheterballone als noch geeignet aber non-compliant Katheterballone als ungeeignet. Die darin offenbarten Wirkstoffabgabesysteme umfassen einen Ballonkatheter mit einem mikroporösen semi-compliant Ballon, der Mikrobohrungen von 0,8 µm - 5 µm aufweisen soll. Tatsächlich waren zum Anmeldezeitpunkt der WO 2012/156914 A2 nur Mikrobohrungen im Bereich von 0,8 µm bis 2,5 µm mit einer Genauigkeit von ±0,5 µm realisierbar. WO 2012/156914 A2 offenbart in Beispiel 1 einen Katheterballon mit Mikrobohrungen von 0,8 µm (bei einer Genauigkeit der Mikrobohrungen von ±0,5 µm) und Beispiel 2 von WO 2012/156914 A2 offenbart einen semi-compliant Katheterballon mit Mikrobohrungen von 1,5 µm (bei einer Genauigkeit der Mikrobohrung von ±0,5 µm) und Beispiel 3 offenbart einen Katheterballon mit Mikrobohrungen von 1,7 µm (bei einer Genauigkeit der Mikrobohrung von ±0,5 µm) und Tabelle 1 von WO 2012/156914 A2 fasst die Versuchsdaten aller getesteter Katheterballone mit Mikrobohrungen im Bereich von 0,8 µm bis 2,5 µm (bei einer Genauigkeit der einzelnen Mikrobohrung von ±0,5 µm) zusammen und es ist offensichtlich, dass Katheterballone mit Mikrobohrungen größer als 2,5 µm fehlen, weil diese zum damaligen Zeitpunkt nicht realisiert werden konnten.

Katheterballone mit Mikrobohrungen im Bereich von 3,0 µm bis 7,5 µm und zudem mit einer Genauigkeit von ±0,3 µm oder noch besser von ±0,2 µm waren zum Anmeldezeitpunkt der WO 2012/156914 A2 nicht herstellbar und somit ist die WO 2012/156914 A2 auch kein Stand der Technik für die vorliegende Erfindung, da die WO 2012/156914 A2 zum Anmeldezeitpunkt für den Bereich von Mikrobohrungen mit Porendurchmessern von 3,0 µm - 7,5 µm nicht ausführbar war, weil derartige Lochgrößen technisch nicht realisierbar waren und vor allem nicht mit einer Genauigkeit von ±0,3 µm oder sogar von ±0,2 µm.

Gemäß WO 2012/156914 A2 lassen sich semi-compliant Ballone nicht mit dem zum Anmeldetag der WO 2012/156914 A2 angewandten "track etching" Verfahren perforieren und wurden mittels Laserstrahlbohren perforiert (s. S. 21 Z 9-12 von WO 2012/156914 A2: *Semi-compliant materials do not lend themselves to perforation by conventional Crack etching processes, and according to some embodiments, are perforated by laser drilling*.). Die Laserstrahlbohrungen haben eine verkürzte konische Form. Hingegen lassen sich non-compliant Ballone mit dem "track etching" Verfahren bohren, wobei der Katheterballon zuvor mit Ionenpartikeln bestrahlt wird (s.S. 32 Z 9-17 von WO 2012/156914 A2). Die Ionenstrahlbohrungen weisen Bohrungsdurchmesser von ∼ 1 µm und weniger auf, hingegen sind die Laserstrahlbohrungen in den semi-compliant Ballonen größer.

Gemäß WO 2012/156914 A2 konnten in 2012 semi-compliant Ballone nicht mit dem "track etching" Verfahren bearbeitet werden. Andererseits konnten non-compliant Ballone nur mit dem track etching" Verfahren bearbeitet werden, aber nicht mittels Laserstrahlen gebohrt werden, so dass in non-compliant Ballone nur Bohrungsdurchmesser von ca. 1 µm realisiert werden konnten. Der Anmelder der vorliegenden Anmeldung hat diese Arbeiten für den Anmelder der WO 2012/156914 A2 in den Jahren 2011 und 2012 durchgeführt und bestätigt die in der WO 2012/156914 A2 gemachten Aussagen.

Beispiel 1 von WO 2012/156914 A2 betrifft aufgrund der Bohrungsdurchmesser von 0,8 µm, welche mittels des track etching" Verfahrens hergestellt wurden und des PET-Materials einen non-compliant Katheterballon. Dieser gemäß WO 2012/156914 A2 nicht erfindungsgemäße non-compliant Katheterballon mit Mikrobohrungen von 0,8 µm und einer Toleranz der Mikrobohrungen von ±0,5 µm ist gemäß Tabelle 1 ungeeignet, die erfindungsgemäße Aufgabe zu lösen, da eine Dilatation über 60 Sekunden bei einem Druck von 15 Atm ein Volumen von nur 0,055 ml freisetzt. WO 2012/156914 A2 hält non-compliant Katheterballone als ungeeignet, weil sich das Ballonmaterial nur ungenügend unter Druck dehnt und bei semi-compliant Katheterballonen unter Druck eine Dehnung der Ballonhülle erfolgt und dadurch auch die Bohrungen gedehnt werden und sich damit deren Durchmesser vergrößert, wodurch mehr Wirkstofflösung freigesetzt wird (s. S. 25, Z. 1-3 von WO 2012/156914 A2). Eine derartige Ausführungsform lässt hingegen keine präzise Messung des Volumens einer freigesetzten Wirkstoffflüssigkeit unter Realbedingungen zu, was aber auch nicht Aufgabe und Ziel der WO 2012/156914 A2 war.

Ferner ist der Tabelle 2 von WO 2012/156914 A2 zu entnehmen, dass bei Drücken von 12 bis 16 atm keine nennenswerte Steigerung des durch die Poren abgegebenen Flüssigkeitsvolumens zu verzeichnen ist, aber in dem für PTCA-Anwendungen üblichen Druckbereich von 16 bis 18 atm eine unkontrollierte Steigerung des applizierten Flüssigkeitsvolumens um einen Zehnerfaktor erfolgt. Aufgrund dieser extremen Änderung des abgegebenen Volumens innerhalb einer moderaten Druckdifferenz von nur 2 atm in dem Druckbereich von 16 atm bis 18 atm ist eine präzise Messung des abgegebenen Flüssigkeitsvolumens pro Zeiteinheit nicht möglich und somit auch die Menge an applizierten Wirkstoff nicht ableitbar. Auch schon aus diesem Grund offenbart die WO 2012/156914 A2 keine Katheterballone mit Bohrungen, welche eine präzise Messung des Volumens einer freigesetzten Wirkstoffflüssigkeit ermöglichen. Dazu offenbart WO 2012/156914 A2 den Vorschlag, diesen rapiden unkontrollierten Anstieg an freigesetzter Flüssigkeit über die Porengröße und/oder die Viskosität der Flüssigkeit möglicherweise kontrollieren zu können. Mehr als diese Idee offenbart die WO 2012/156914 A2 jedoch nicht, so dass es an einer technischen Lehre zur Lösung dieses Problems mangelt.

Die Erfinder haben zur Herstellung von mikrogebohrten Katheterballons bereits im Zeitraum der Offenlegung von WO 2012/156914 A2 und US 2011/160575 A1 Untersuchungen durchgeführt siehe (Beispiel 3 und Figuren 8 bis 11).

Die Wirkstoffabgabesysteme mit einem Ballonkatheter mit einem mikroporösen Ballon, der Mikrobohrungen von 0,8 µm - 5 µm aufweist, konnten jedoch von den Erfindern mit den in WO 2012/156914 A2 und US 2011/160575 A1 beschriebenen Verfahren und Vorrichtungen zur Herstellung mikroporöser Katheterballons nicht zeichnungsgerecht, reproduzierbar und nicht mit der für den Einsatz in der erfindungsgemäßen Inflationsvorrichtung (1) notwendigen Präzision hergestellt werden. Erst recht konnten zum Anmeldetag der WO 2012/156914 A2 keine non-compliant Ballone mit Mikrobohrungen zur präzisen Abgabe eines Flüssigkeitsvolumens mit Bohrungsdurchmessern von 3,0 µm bis 7,5 µm hergestellt werden.

Im einschlägigen Stand der Technik werden zwei Verfahren zur Erzeugung von Mikrobohrungen in Katheterballone beschrieben: Das Ionenstrahlverfahren für Mikrobohrungen mit einem Durchmessern < 2,5 µm und das Laserstrahlbohren mittels Ultrakurzpuls-Laser (UKP-Laser).

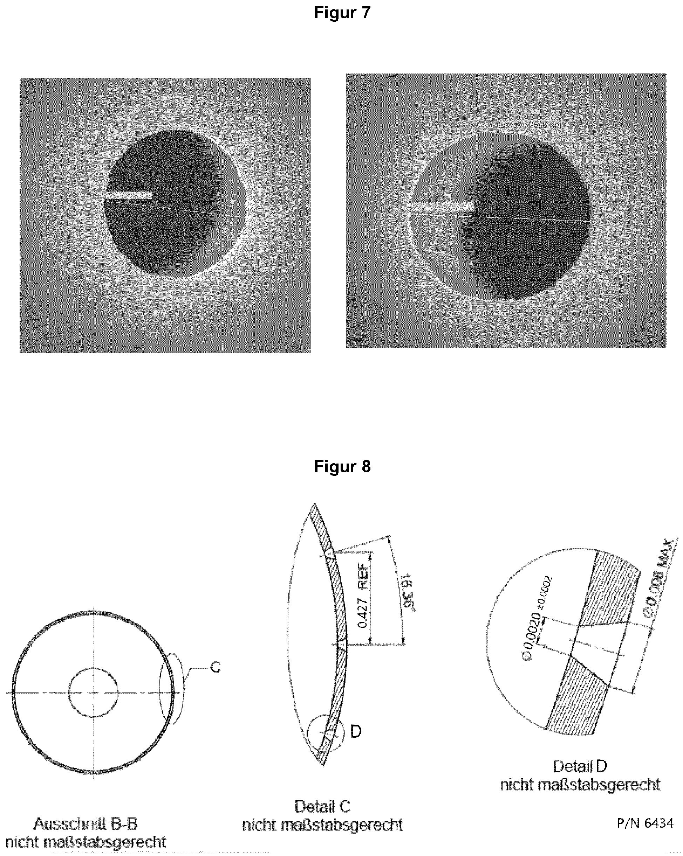

Beim Ionenstrahlverfahren entstehen Bohrungen in den Katheterballons mit Durchmessern kleiner 2,5 µm, vorzugsweise 1,7 µm, durch Ionenbestrahlung mit einem Zyklotron und anschließendem chemischen Ätzen (siehe Figur 7). Die Ionenstrahlen bzw. Ionenteilchen durchdringen die Kunststoffwand des Ballons und spalten die Moleküle entlang des Durchdringungskanals, so dass durch das anschließende chemische Ätzen winzige Kanäle in der Ballonwand entstehen. Mit diesem Verfahren können kleinste Bohrungen mit einem Durchmesser von 0,8 µm erzeugt werden.

Eine gezielte Vergrößerung der Bohrungsdurchmesser ist mit dem Ionenstrahlverfahren nicht möglich, da die Ionen eine feste Größe/Durchmesser haben und auch keine Fokussierung mehrerer Ionenteilchen erfolgt, sondern der Ballon des Katheters einem "Bombardement" einzelner Teilchen ausgesetzt ist. Mit dem Ionenstrahlverfahren sind folglich keine Bohrungen im Ballonkatheter realisierbar, die größer als 2,5 µm sind (siehe Figur 7).

Darüber hinaus weist das lonenstrahlbohrverfahren weitere Einschränkungen auf: Die Anzahl der Bohrungen kann nur über die Dauer des Ionenbeschusses eingestellt werden. Eine singuläre Erzeugung einzelner Bohrungen ist somit nicht möglich. Die Positionierung einzelner Bohrungen auf dem Ballon ist ebenfalls nicht möglich, da die Verteilung der Bohrungen stochastisch ist. Lediglich durch Abschattung des Ionenstrahls können Bohrbereiche auf dem Ballon definiert werden. Die Ionenstrahlbohrungen sind somit undefinierbar und eine zuverlässig reproduzierbare Wirkstoffabgabe aus seriell gebohrten Ballons nicht möglich.

Der entscheidende Nachteil des Ionenstrahlverfahrens ist aber die anschließende chemische Ätzung zum Öffnen der Mikrobohrungen. Dadurch dringt Säure in den Katheterballon, die sich weder durch die Ballonbohrungen noch durch den Katheterschlauch ausspülen lässt. Durch diese Kontamination des Balloninneren wird der Katheterballon für den Einsatz im menschlichen Körper unbrauchbar.

Das im Stand der Technik beschriebene Ionenstrahlverfahren hat sich, aufgrund der zu kleinen Bohrungen von maximal 2,5 µm im Durchmesser und der Kontamination des Ballons durch die chemische Ätzung, als ungeeignet für die mit der erfindungsgemäßen Inflationsvorrichtung (1) vorgesehene Verwendung im menschlichen Körper herausgestellt. Mittels Ionenstrahlverfahren hergestellte Katheterballons sind damit ungeeignet für die Verwendung in der hierin beschriebenen erfindungsgemäßen Inflationsvorrichtung (1).

Aufgrund der genannten Nachteile und Limitierungen des Ionenstrahl-Bohrverfahrens wurde alternativ das **Laserstrahlbohren** der Katheterballone untersucht, wobei Katheterballone aus PET und Nylon verwendet wurden.

Jedoch war die Herstellung von präzisen und reproduzierbaren Mikrobohrungen (ca. 2 µm Durchmesser) mit dem im Stand der Technik WO 2012/156914 A2 aus dem Jahr 2012 beschriebenem Laserbohrverfahren und der Laserbohrvorrichtung aufgrund von technischen Limitierungen des Lasersystems nicht realisierbar. Konkret war die Pulsdauer der UKP-Laser und die Tiefenschärfe im Strahlfokus unzureichend sowie die Laserstrahlqualität eingeschränkt. Zudem waren Fokussieroptiken und Frequenzkonversion des Laserstrahls nicht in der Lage, den Laserstrahl auf einen ausreichend kleinen Durchmesser zu reduzieren und die verwendete Maschinen- und Prozesstechnik nicht präzise genug.

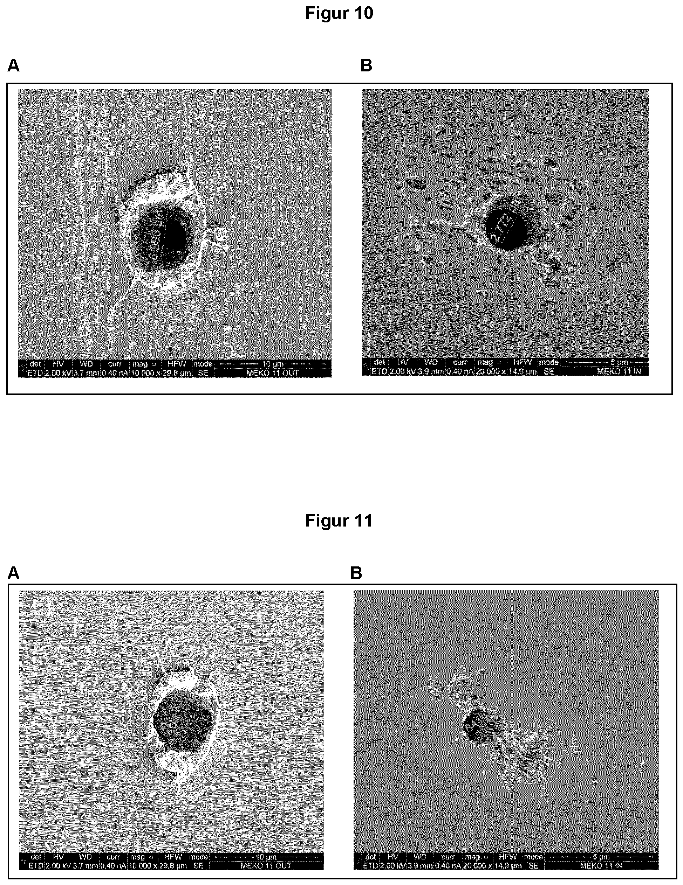

Die verfügbaren UKP-Laser hatten Pulsdauern im Pikosekundenbereich, was zu einem signifikanten Wärmeeintrag und in Folge zu unerwünschten Schmelzzonen sowie Materialaufwürfe führte (siehe Figur 10A). Kürzere Pulsdauern, idealerweise im Femtosekundenbereich, sind daher erforderlich, um die Wärmeeinleitung zu minimieren und unerwünschte Schmelzzonen sowie Materialaufwürfe im Katheterballon zu vermeiden. Mikrobohrungen im Durchmesserbereich von wenigen Mikrometern, zudem in Kunststoffen, lassen sich nur mit ultrakurzen Laserpulsen von kürzer als 100 ps (Pikosekunden) herstellen.

Die hohen Pulsleistungen der damals verfügbaren UKP-Laser erzeugten zusätzliche höhere Modenordnungen, die den Laserstrahl verbreiterten und dessen Fokussierbarkeit einschränkten. Zwar konnte durch Frequenzkonversion (z B. Erzeugung von 515 nm oder 343 nm Wellenlängen) die theoretische Auflösung verbessert werden, jedoch führten zusätzliche optische Elemente (Kristalle) zu Aberrationen und Leistungsverlusten. Die begrenzte Strahlqualität des UKP-Laserstrahls zeigt insbesondere Figur 10B. Um die zentrale Bohrung herum sind Materialschäden zu erkennen, die durch den aufgefächerten Laserstrahl verursacht wurden. Aufgrund des aufgefächerten Laserstrahls wurde mit der grünen Wellenlänge von 515 nm und nicht mit der (besser fokussierbaren) ultravioletten Wellenlänge von 343 nm gebohrt, da UV-Strahlung Kunststoffverbindungen crackt und das Ballonmaterial schädigt. Die Bereiche um die eigentliche Laserbohrung wurden durch die diffuse UV-Laserstrahlung so geschädigt, dass die Bohrungen bei hohen Ballondrücken aufrissen. Eine verbesserte Strahlqualität ist somit unerlässlich, um den Strahl nahezu parallel und mit hoher Tiefenschärfe zu fokussieren, damit Mikrobohrungen im Bereich von ca. 2 µm erzeugt werden können. Nur so kann ein nahezu idealer Fokus, theoretisch nahe am physikalischen Limit der Wellenlänge (ca. 1 µm), erreicht werden. Die verfügbaren Fokussieroptiken waren somit nicht in der Lage, den Laserstrahl auf einen ausreichend kleinen Durchmesser zu reduzieren, ohne dass es zu Streuungen und ungleichmäßigen Bohrungen kam. Präzisere und verzehrungsfreie Optiken sind daher nötig, um den Laserstrahl ohne Qualitätsverlust auf einen sehr kleinen Durchmesser zu fokussieren.

Die Bewegungstische und Strahlführungssysteme verfügten nur über eine Genauigkeit von ±0,5 µm, was im Vergleich zur minimal erforderlichen Tiefenschärfe im Fokus (um konstant 2 µm zu bohren) zu erheblichen Schwankungen in den Bohrungsdurchmessern, insbesondere angesichts der variierenden Wandstärken der Katheterballons. Präzisere Steuerungen mit einer Genauigkeit im Submikrometerbereich (ideal < ±0,1 µm) und adaptive Prozessstrategien, die Wandstärkenschwankungen kompensieren können, sind jedoch unabdingbar.

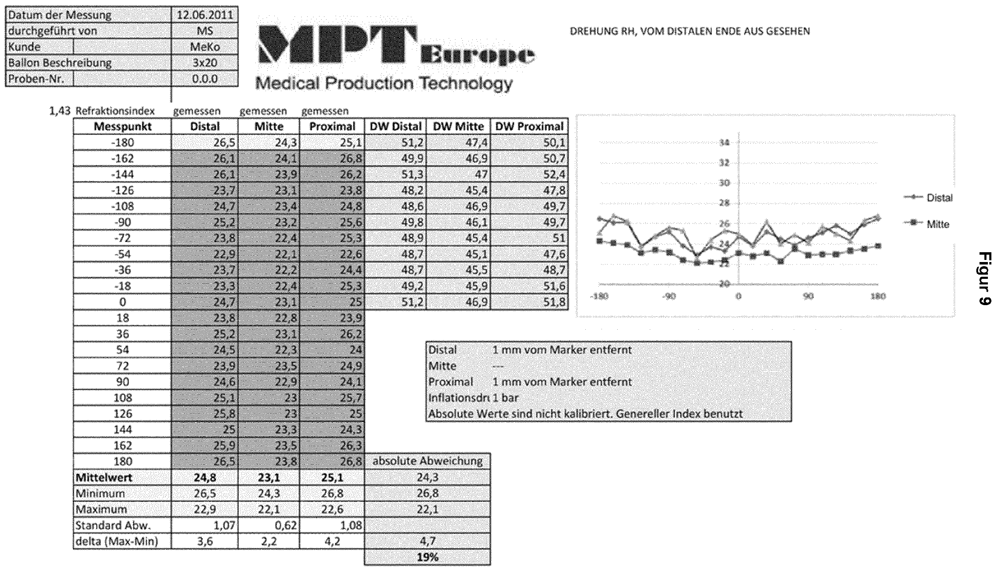

Zum Zeitpunkt der Offenbarung von WO 2012/156914 A2 wiesen Katheterballons üblicherweise eine mittlere Wandstärke von ca. 24 µm (siehe Figur 9) auf. Im Fall von Bohrungen mit etwa 2 µm Durchmesser, folgt daraus ein Verhältnis von über 1:10, was bedeutet, dass der Laserstrahl über eine 24 µm dicke Wand einen konstanten 2 µm-Durchmesser beibehalten muss. Dies erfordert eine extrem hohe Tiefenschärfe (Rayleighlänge) im Strahlfokus, die zum Zeitpunkt der Offenbarung von WO 2012/156914 A2 technisch noch nicht erfüllt werden konnte.

Auch die Wandstärkenschwankungen auf einem Katheterballon (siehe Figur 9) von ± 2 µm erschweren das Laserbohren im µm-Bereich. Wenn der Bohrungsdurchmesser unabhängig von Wandstärkeschwankungen konstant sein soll, muss ein nahezu paralleler Strahl im Strahlfokus vorliegen, d.h. es ist eine hohe Tiefenschärfe im Fokus gefordert. Die hierzu notwendige Strahlqualität konnte jedoch mit dem in WO 2012/156914 A2 beschriebenen Lasersystem aus den Jahren 2010 bis 2012 nicht erreicht werden.

Aufgrund dieser technologischen Beschränkungen (unzureichende Tiefenschärfe im Strahlfokus, mangelhafte Strahlqualität, thermische Effekte durch zu lange Pulsdauern und ungenaue Maschinensteuerungen) war es der Fachperson anhand der Offenbarung von WO 2012/156914 A2 nicht möglich, Katheterballons mit Mikrobohrungen in der erforderlichen Präzision und Reproduzierbarkeit herzustellen. Zum Zeitpunkt der Offenbarung von WO 2012/156914 A2 waren diese notwendigen Prozessparameter noch nicht verfügbar und daher das Laserbohrverfahren in der erforderlichen Qualität nicht umsetzbar.

Um dennoch kleinste Mikrobohrungen mit einem Durchmesser von 2 µm (siehe Figur 8) zu erzeugen, wurden bisher **konische Bohrungen** in die Ballonwand gelasert. Die Erfinder selbst hatten hierzu Untersuchungen im selben Zeitraum der Offenbarung von WO 2012/156914 A2 durchgeführt (siehe Figuren 8 bis 11). Gemäß Zeichnung in Figur 8 sollten die Bohrungen 2,0 ± 0,2 µm groß sein.

Bei diesem Verfahren des Laserbohrens konischer Bohrungen werden die Prozessparameter so eingestellt, dass im Moment der ersten vollständigen Durchdringung der Wandung die Bearbeitung abgebrochen wurde. Bei diesem Verfahren mit Abbruch der Bearbeitung nach einer vorbestimmten Zeit führen jedoch Schwankungen der Ballonwanddicke zu signifikant unterschiedlichen Bohrungsdurchmessern. Bei dickeren Wandstärken wird nicht durchgebohrt, bei dünneren Wandstärken wird zu groß gebohrt. Die Bohrungsdurchmesser streuen um mehr als 1 µm im Durchmesser, was bei Sollwerten von ~2 µm zu groß ist (siehe Figur 10 und 11). Die Figuren 10 und 11 zeigen exemplarisch konische Laserstrahlbohrungen. Der Durchmesser der Bohrungen auf der Ballonaußenseite (Laserstrahl-Eintrittsseite) beträgt ~7 µm und auf der Balloninnenseite (Laserstrahl-Austrittsseite) 1,8 µm bzw. 2,7 µm. In den Figuren 10 und 11 sind auf der Laserstrahl-Eintrittsseite Materialaufwürfe rings um die Bohrung zu sehen. Auf der Laserstrahl-Austrittsseite sind um die Bohrung diffuse Lochmuster zu sehen, was auf eine Auffächerung eines nicht optimal fokussierten Laserstrahls schließen lässt.

In Folge der oben beschriebenen Verfahrensoptimierung und Prozessstabilisierung beim Laserstrahlbohren (siehe Beispiel 4) konnten die Erfinder Anfang 2025 Bohrungstoleranzen von unter 5% erzielen. Dabei können nun Bohrungen mit einem Durchmesser von 5,0 µm mit einer **Genauigkeit von ±0,2 µm** hergestellt werden. Dies entspricht einer Durchmessertoleranz von ±4%. Überraschenderweise stellten die Erfinder in diesem Zusammenhang fest, dass für eine zuverlässige und reproduzierbare Wirkstoffabgabe mikrogebohrte Katheterballons mit einer **Toleranz** oder Maßgenauigkeit der Mikrobohrungen im Durchmesser von weniger als 5% besonders vorteilhaft sind.

Unter **Toleranz** wird im Rahmen dieser Patentanmeldung der maximal zulässige prozentuale Abweichungsbereich der tatsächlichen Bohrungsdurchmesser von einem definierten Sollwert verstanden. Konkret bedeutet dies, dass die beim Laserbohren erzeugten Mikrobohrungen des Katheterballons in ihrem Durchmesser so reproduzierbar sein müssen, dass die Differenz zwischen dem Soll- und dem IstDurchmesser eine vorgegebene Grenze (z.B. 5 %) oder weniger beträgt. Diese Toleranzgrenze stellt sicher, dass die Funktionalität und die gleichmäßige Verteilung der Bohrungen zur gewünschten Wirkstoffabgabe bzw. mechanischen Stabilität des Katheterballons gewährleistet ist.

Die im Stand der Technik (z.B. WO2012/156914A2) erreichte Durchmessertoleranz von 0,5 µm ist für eine zuverlässige und reproduzierbare Wirkstoffabgabe mit mikrogebohrten Katheterballons zu groß, insbesondere wenn die Bohrungen einen Durchmesser zwischen 1 µm und 5 µm aufweisen. Bei Bohrungen von 1 µm und einer Toleranz von 0,5 µm ist eine 9-fache Querschnittsflächenänderung der Mikrobohrung möglich. Bei einem Durchmesser von 5 µm liegt immer noch eine Querschnittsänderung von 50% und damit eine zu hohe Varianz der Wirkstoffabgabe vor.

In einer bevorzugten Ausführungsform umfasst die erfindungsgemäße Vorrichtung (1) bzw. Inflationsvorrichtung (1) für Mikrobohrungen (11) aufweisende non-compliant Katheterballone (10) zur dosierten Freisetzung einer Wirkstoffflüssigkeit (20) daher
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen (11) mit einem Bohrungsdurchmesser im Bereich von 3,0 µm bis 7,5 µm, bevorzugt 3,0 µm bis 7,0 µm, bevorzugt von 3,5 µm bis 6,5 µm, weiter bevorzugt von 4,0 µm bis 6,0 µm und mit einer Toleranz der Mikrobohrungen (11) im Durchmesser von maximal 5% und/oder einer Genauigkeit der Durchmesser der Mikrobohrungen (11) von ±0,2 µm aufweist.

In einer weiteren bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion** (21) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion (21) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion (21) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion (21) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion (21) anzuzeigen,
wobei der Katheterballon (10) Mikrobohrungen (11) mit einem Bohrungsdurchmesser im Bereich von 3,5 µm bis 7,5 µm oder 4,0 µm bis 7,5 µm, bevorzugt von 4,0 µm bis 7,0 µm, bevorzugt von 4,5 µm bis 7,5 µm, bevorzugt von 4,5 µm bis 7,0 µm, weiter bevorzugt von 5,0 µm bis 7,5 µm, bevorzugt von 5,0 µm bis 7,0 µm, weiter bevorzugt von 5,5 µm bis 7,5 µm und mit einer Toleranz der Mikrobohrungen (11) im Durchmesser von maximal 5% und/oder einer Genauigkeit der Durchmesser der Mikrobohrungen (11) von ±0,2 µm aufweist.

In einer anderen bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Mikrospheren** (22) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Mikrospheren (22) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Mikrospheren (22) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Mikrospheren (22) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Mikrospheren (22) anzuzeigen,
wobei der Katheterballon (10) Mikrobohrungen (11) mit einem Bohrungsdurchmesser im Bereich von 3,5 µm bis 7,5 µm oder 4,0 µm bis 7,5 µm, bevorzugt von 4,5 µm bis 7,5 µm, weiter bevorzugt von 5,0 µm bis 7,5 µm, weiter bevorzugt von 5,5 µm bis 7,5 µm und mit einer Toleranz der Mikrobohrungen (11) im Durchmesser von maximal 5% und/oder einer Genauigkeit der Durchmesser der Mikrobohrungen (11) von ±0,2 µm aufweist.

Die Mikrospheren (22) haben normalerweise einen Durchmesser von wenigen Mikrometern wie z.B. im Bereich vom 3 µm bis 5 µm.

Ferner betrifft die vorliegende Erfindung eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Mikrospheren** (22) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Mikrospheren (22) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Mikrospheren (22) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Mikrospheren (22) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Mikrospheren (22) anzuzeigen,
wobei der Katheterballon (10) Mikrobohrungen (11) mit einem Bohrungsdurchmesser im Bereich von 3,5 µm bis 7,5 µm oder von 4,0 µm bis 7,5 µm, oder von 4,5 µm bis 7,5 µm, oder von 5,0 µm bis 7,5 µm, oder von 5,5 µm bis 7,5 µm und mit einer Toleranz der Mikrobohrungen (11) im Durchmesser von maximal 5% und/oder einer Genauigkeit der Durchmesser der Mikrobohrungen (11) von ±0,2 µm aufweist und die Mikrobohrungen (11) einen um mindestens 0,5 µm oder mindestens 0,6 µm oder mindestens 0,7 µm oder mindestens 0,8 µm größeren Durchmesser als die Mikrospheren (22) haben.

In einer anderen bevorzugten Ausführungsform betrifft die vorliegende Erfindung daher eine Vorrichtung (1), bzw. präziser eine Inflationsvorrichtung (1) für Mikrobohrungen (11) aufweisende non-compliant Katheterballone (10) zur dosierten Freisetzung einer **Wirkstoffdispersion von Nanospheren** (23) umfassend
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Nanospheren (23) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Nanospheren (23) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Nanospheren (23) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffdispersion von Nanospheren (23) anzuzeigen,
wobei der Katheterballon (10) Mikrobohrungen (11) mit einem Bohrungsdurchmesser im Bereich von 3,0 µm bis 7,5 µm, bevorzugt von 3,5 µm bis 6,5 µm, weiter bevorzugt von 4,0 µm bis 6,0 µm und mit einer Toleranz der Mikrobohrungen (11) im Durchmesser von maximal 5% und/oder einer Genauigkeit der Durchmesser der Mikrobohrungen (11) von ±0,2 µm aufweist.

Vorzugsweise beträgt die erreichte Durchmessertoleranz 4% oder weniger.

In einer anderen Ausführungsform umfasst die erfindungsgemäße Vorrichtung (1) bzw. Inflationsvorrichtung (1) für Mikrobohrungen (11) aufweisende non-compliant Katheterballone (10) zur dosierten Freisetzung einer **Wirkstoffflüssigkeit** (20)
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen mit einem Bohrungsdurchmesser im Bereich von 3,0 µm ±0,2 µm bis 7,5 µm ±0,2 µm, bevorzugt von 3,5 µm ±0,2 µm bis 6,5 µm ±0,2 µm, weiter bevorzugt von 4,0 µm ±0,2 µm bis 6,0 µm ±0,2 µm aufweist.

Vorzugsweise beträgt die Durchmessertoleranz ±0,2 µm. In einer weiteren Ausführungsform liegt die Durchmessertoleranz zwischen ±0,1 µm und ±0,3 µm, vorzugweise zwischen ±0,11 µm und ±0,29 µm, vorzugweise zwischen ±0,12 µm und ±0,28 µm, vorzugweise zwischen ±0,13 µm und ±0,27 µm, vorzugweise zwischen ±0,14 µm und ±0,26 µm, weiter vorzugweise zwischen ±0,15 µm und ±0,25 µm, weiter vorzugweise zwischen ±0,16 µm und ±0,24 µm, weiter vorzugweise zwischen ±0,17 µm und ±0,23 µm, weiter vorzugweise zwischen ±0,18 µm und ±0,22 µm und am meisten bevorzugt zwischen ±0,19 µm und ±0,21 µm.

Vorzugsweise umfasst die erfindungsgemäße Vorrichtung (1) bzw. Inflationsvorrichtung (1) für Mikrobohrungen (11) aufweisende non-compliant Katheterballone (10) zur dosierten Freisetzung einer **Wirkstoffflüssigkeit** (20)
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen (11) mit einem Bohrungsdurchmesser im Bereich von 3,0 µm bis 7,5 µm oder 3,0 µm bis 7,0 µm, bevorzugt von 3,5 µm bis 6,5 µm, weiter bevorzugt von 4,0 µm bis 6,0 µm und mit einer Toleranz der Mikrobohrungen (11) im Durchmesser von maximal 5% und/oder einer Genauigkeit der Durchmesser der Mikrobohrungen (11) von ±0,2 µm aufweist und wobei nach dreifacher Dilatation oder nach fünffacher Dilatation die irreversible Weitung der Mikrobohrungen weniger als 5% des Bohrungsdurchmessers, bevorzugt weniger als 4% des Bohrungsdurchmessers, weiter bevorzugt weniger als 3% des Bohrungsdurchmessers, weiter bevorzugt weniger als 2,5% des Bohrungsdurchmessers, weiter bevorzugt weniger als 2% des Bohrungsdurchmessers beträgt.

Vorzugsweise umfasst die erfindungsgemäße Vorrichtung (1) bzw. Inflationsvorrichtung (1) für Mikrobohrungen (11) aufweisende non-compliant Katheterballone (10) zur dosierten Freisetzung einer Wirkstoffflüssigkeit (20)
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffflüssigkeit (20) freigesetzt wird, zu erfassen,
- eine Recheneinheit (5), die konfiguriert ist, das vom Katheterballon (10) durch die Mikrobohrungen (11) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen,
wobei der Katheterballon (10) Mikrobohrungen (11) mit einem Bohrungsdurchmesser im Bereich von 3,0 µm bis 7,5 µm oder 3,0 µm bis 7,0 µm, bevorzugt von 3,5 µm bis 6,5 µm, weiter bevorzugt von 4,0 µm bis 6,0 µm und mit einer Toleranz der Mikrobohrungen (11) im Durchmesser von maximal 5% und/oder einer Genauigkeit der Durchmesser der Mikrobohrungen (11) von ±0,2 µm aufweist und die Anzahl der Mikrobohrungen auf der Ballonoberfläche 0,3 bis 4,0 Mikrobohrungen pro mm², bevorzugt 1,0 - 3,0 Mikrobohrungen pro mm², besonders bevorzugt 1,5 bis 2,5 Mikrobohrungen pro mm² beträgt.

Stenosen in Blutgefäßen bestehen häufig aus Kalzifizierungen. Diese Kalzifizierungen lassen sich aufgrund ihrer Festigkeit und Härte nur schwer mit einem Ballon aufdehnen. Häufig sind hohe Ballondrücke erforderlich, um die Kalzifizierung aufzubrechen. Insbesondere bei auf dem Gefäßumfang einseitigen Kalzifizierungen führt eine reine Ballondilatation - ohne Einsatz eines Stents - zu einseitigen, asymmetrischen Gefäßweitungen, d.h. die gesunde, nicht kalzifizierte Gefäßwandung wird übermäßig gedehnt, verbunden mit dem hohen Risko von Dissektionen und Gefäßeinrissen.

Um Kalzifizierungen einfacher aufzubrechen und vor allem für eine gleichmäßigere Dehnung der kalzifizierten Stenosen werden sogenannte Cutting Ballone oder Scoring Ballone (Philips/Spectralytics, Teleflex/GVascular/Trireme/Quattro) verwandt. Bei den **Cutting Ballonen** sind auf der Ballonoberfläche längliche Klingen in axialer Richtung befestigt, die die Kalzifizierung einschneiden und damit aufbrechen sollen. Bei den **Scoring Ballonen** wird der Ballon mit einer netzartigen Struktur überzogen, die aus mehreren dünnen elastischen Stegen besteht, wobei die Stege meist spiralförmig auf dem Ballonumfang angeordnet sind. Die Stege sollen lokal Kräfte auf die Kalzifizierung ausüben und somit zum Aufbrechen der Kalzifizierung beitragen.

Bei einer bevorzugten patentgemäßen Ausgestaltung der Cutting Ballone und Scoring Ballone sind diese Ballone mit Mikrobohrungen versehen. Im Moment des Aufbrechens der Kalzifizierungen wird die Gefäßwandung unmittelbar mit einer Wirkstoffflüssigkeit versorgt. Insbesondere wird der Wirkstoff in die aufgerissenen Spalte der Kalzifizierung gepresst, womit eine optimale und sehr wirksame Versorgung des Gefäßes mit Wirkstoff erreicht wird.

Bei erfindungsgemäßer Ausgestaltung sind auf dem Umfang der Cutting bzw. Scoring Ballone Mikrobohrungen mit einem Durchmesser von 3,0 µm bis 7,5 µm oder 3,0 µm bis 7,0 µm, bevorzugt von 3,5 µm bis 6,5 µm, weiter bevorzugt von 4,0 µm bis 6,0 µm gebohrt. Der Operateur kann nach Aufbrechen der Kalzifizierung die Stenose mit Wirkstoff versorgen, wobei er die Wirkstoffmenge über den Flüssigkeitsdruck und die Zeitdauer der Ballondehnung steuert. Hierzu nutzt er die erfindungsgemäße Vorrichtung zur kontrollierten und dosierten Abgabe von Wirkstoffflüssigkeiten.

Eine weitere bevorzugte patentgemäße Ausgestaltung der Drossel ist ein mit Mikrobohrungen versehener **Lithotripsie Ballonkatheter** z.B. von der Firma Shockwave Medical (https://shockwavemedical.com) oder FastWave Medical (https://www.fastwavemedical.com) oder Bolt Medical (https://boltmed.com/) oder Vantis Vascular (https://vantisvascular.com) und Amplitude Vascular (https://www.avspulse.com) zur intravaskulären Lithotripsie von kalzifizierten Blutgefäßen. Bei dieser Art von speziellen Lithotripsie Ballonkatheter sind in dem Ballon des Katheters winzige Emitter integriert, die beim elektrischem Impuls Schallwellen so hoher Intensität aussenden, dass harte Blutgefäß-Kalzifizierungen aufbrechen. Derzeit werden die Lithotripsie Ballonkatheter nur mit geringem Überdruck (z.B. ca. 4 bar) aufgedehnt, damit sie an der kalzifizierten Gefäßwandung anliegen, bevor die Schallwellenanwendung gestartet wird.

Bei erfindungsgemäßer Ausgestaltung wird der Ballon des Lithotripsie-Katheters mit Mikrobohrungen mit Durchmessern 3,0 µm bis 7,5 µm oder 3,0 µm bis 7,0 µm, bevorzugt von 3,5 µm bis 6,5 µm, weiter bevorzugt von 4,0 µm bis 6,0 µm versehen. Im Moment, wo die Schallwellen die harten Kalzifizierungen aufbrechen, strömt die Wirkstoffflüssigkeit in Bruchstellen der Kalzifizierungen und auch in die mit verletzte Gefäßwandung. Die Schallwellen führen auch dazu, dass die Wirkstoffflüssigkeit besser in die Gefäßwandung hineindiffundiert. Dieser Effekt tritt insbesondere auf, wenn die Wirkstoffflüssigkeit als Dispersion vorliegt mit festen Wirkstoffpartikeln, die von den Schallwellen angeregt werden.

Über die erfindungsgemäße Vorrichtung zur kontrollierten und dosierten Abgabe kann die abgegebene Flüssigkeitsmenge während der Schallwellenbehandlung, aber auch davor und insbesondere danach genau gesteuert werden. Eine kalzifizierte Stenose eines Blutgefäßes kann auf diese Weise optimal intravaskulär mechanisch und medikamentös behandelt werden.

Eine weitere bevorzugte patentgemäße Anwendung eines mikrogebohrten Katheterballons mit kontrollierter und dosierter Wirkstoffabgabe sind selbstexpandierende **Stents mit selbstexpandierenden Spikes** der Firma Reflow Medical (https://www.reflowmedical.com/spur/). Das in klinischen Studien bestätigte Konzept des sogenannten "SPUR" Stents ist, die Gefäßwandung mit einer Vielzahl von kleinen Spikes einzustechen, um eine bessere Zufuhr von Medikamenten in die Gefäßwandung zu erreichen. Die Medikamentenbehandlung erfolgt aber nur entweder durch eine Oberflächenbeschichtung des SPUR-Stents oder durch eine anschließende Behandlung mit einem Drug Coated Balloon (DCB). Beide Formen der Medikamentenbehandlung sind von der Dosis und der Medikamentenzufuhr sehr limitiert.

Bei der Anwendung eines mikrogebohrten Katheterballons mit erfindungsgemäßer kontrollierter und dosierter Wirkstoffabgabe wird der SPUR-Stent mit dem mikrogebohrten Katheterballon aufgedehnt. In die von den Spikes vielfach eingestochene Gefäßwandung wird die Wirkstoffflüssigkeit abgegeben. Auf diese Weise erfolgt eine sehr wirksame Wirkstoffversorgung der Gefäßwandung, wobei die Dosis und Dauer der Wirkstoffabgabe in der Anzeigeeinheit (Display) der Recheneinheit angezeigt wird und vom Operateur gesteuert werden kann. Ein mehrfaches Schließen und Öffnen des SPUR-Stents und der Wirkstoffabgabe an die Gefäßwandung ist möglich.

### Figurenbeschreibung

- **Figur 1**:: Eine Ausführungsform der erfindungsgemäßen Vorrichtung zur kontrollierten und dosierten Abgabe von Wirkstoffflüssigkeiten im Körperinneren, bei der alle Komponenten der Vorrichtung separat voneinander getrennt sind.
- **Figur 2:**: zeigt (**A**) Korrelationskurven für die Durchflussrate in Abhängigkeit der Flüssigkeitsdrücke und drei verschiedene Bohrungsanzahlen in der Ballonwandung, und (**B**) Korrelationskurven für die Durchflussrate für semi- und non-compliant Katheterballone in Abhängigkeit der Flüssigkeitsdrücke für verschiedene Bohrungsanzahlen.
- **Figur 3:**: Apparatur zur präzisen Wirkstoffvolumenmessung mit Pumpeinheit und separatem Drucksensor und separaten Recheneinheit samt Anzeigeeinheit (Display).
- **Figur 4:**: Pumpeinheit mit integriertem Drucksensor (3), Recheneinheit (5) und Anzeigeeinheit/Display (6).
- **Figur 5:**: Pumpeinheit mit integriertem Drucksensor und integrierter Recheneinheit samt Anzeigeeinheit (Display) zur präzisen Wirkstoffvolumenmessung.
- **Figur 6:**: Apparatur zur Messung der Durchflussraten (hier für Wasser als Flüssigkeit).
- **Figur 7:**: Ionenstrahlbohrungen in Katheterballone.
- **Figur 8:**: Ausschnitt aus der technischen Zeichnung für einen lasergebohrten Katheterballons.
- **Figur 9:**: Wandstärkenmessung eines Katheterballons.
- **Figur 10:**: zeigt Aufnahmen der Laserstrahleinstritts- (**A**) und -austrittsseite (**B**) einer Katheter-Ballonwandung.
- **Figur 11:**: zeigt Aufnahmen der Laserstrahleinstritts- (**A**) und -austrittsseite (**B**) einer Katheter-Ballonwandung.
- **Figur 12:**: zeigt Aufnahmen der Laserbohrung in eine Katheterballonwandung mit Durchmesser 4,6 µm (**A**) und4,89 µm (**B**) (Laserstrahleintrittsseite).
- **Figur 13:**: zeigt Aufnahmen von Dissektionen der Gefäßwandung von Koronargefäßen durch zu hohe Flüssigkeitsabgabe von Katheterballonen mit Ballonbohrungen größer 8 µm

### Beispiele

### Beispiel 1: Beispielhafte Durchführung einer Gefäßbehandlung (Stenose) mit der erfindungsgemäßen Vorrichtung (1)

1. Vorbereitung der erfindungsgemäßen Vorrichtung (1)
   a) Auswahl der Korrelationskurve
   b) Anschließen des Ballonkatheters mit Katheterballon an die Vorrichtung
   c) Aufnahme der Wirkstoffflüssigkeit in die Pumpeinheit, z.B. Inflator der Vorrichtung
   d) Einstellen der passenden Korrelationskurve und des Schwelldruckes für die jeweilige Wirkstoffflüssigkeit und den jeweiligen Ballonkatheter an der Recheneinheit der Vorrichtung
2. Durchführung der Behandlung
   a) Einführen des Ballonkatheters und Positionierung an dem zu behandelnden Gefäßabschnitt
   b) Inflation des Katheterballons mittels der erfindungsgemäßen Vorrichtung
   c) Ablesen der abgegebenen Wirkstoffmenge, des Flüssigkeitsdruckes und der Behandlungszeit
   d) Deflation des Katheterballons
   e) ggf. Wiederholen der Schritte b) bis d) zur wiederholten Behandlung des Gefäßabschnitts

### Beispiel 2: Bestimmung der Korrelationskurve (Figur 2A und 2B)

Die Durchflussraten durch eine Drossel z.B. einen mikrogebohrten Ballonkatheter sind sehr gering. Zur Bestimmung der Durchflussraten von Flüssigkeiten sind 2 Verfahren einsetzbar:
Bei konstant gehaltenem Flüssigkeitsdruck wird für einen längeren Zeitdauer die gesamte abgegebene Flüssigkeitsmenge gemessen. Die Zeitdauer ist so zu bemessen, dass eine nennenswerte messbare Flüssigkeitsmenge abgegeben wird, z.B. 20 ml. Aus gemessener Flüssigkeitsmenge und Zeitdauer ergibt sich die Durchflussrate ml/s für den entsprechenden Flüssigkeitsdruck und die Viskosität der Flüssigkeit. Diese Messungen sind für jeden Flüssigkeitsdruck in Abhängigkeit der Viskosität der Flüssigkeit zu wiederholen.

Dieses Verfahren ist aufwändig und fehlerbehaftet, da über eine längere Zeitdauer abgegebene Flüssigkeiten zu Verstopfungen der Drossel respektive der Mikrobohrungen führen können.

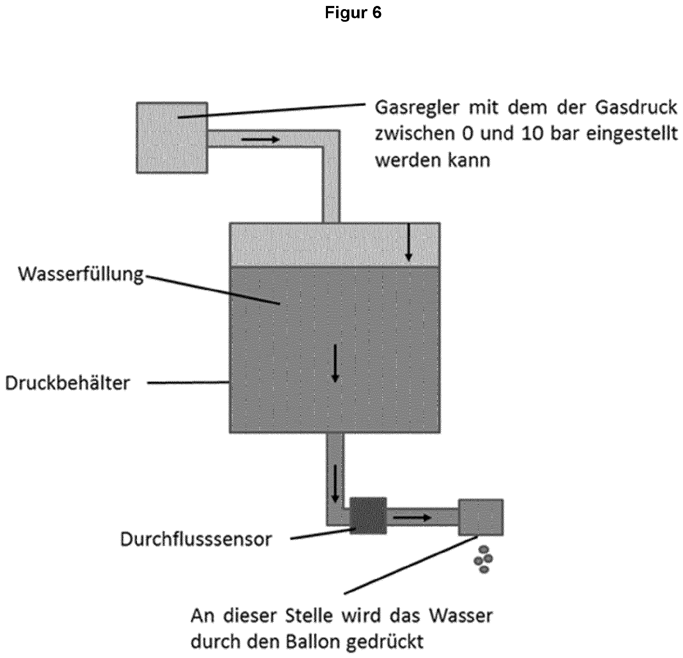

Deshalb wurde eine Messapparatur mit einem speziellen Durchfluss-Sensor entwickelt, der kleinste Durchflussraten von µl/s präzise erfassen kann (Figur 6). Diese Apparatur zeigt online zu jedem Zeitpunkt die Durchflussrate pro Sekunde und den Flüssigkeitsdruck an. Dadurch lassen sich die Korrelationskurven schnell und verlässlich für jede Drossel, Wirkstoffflüssigkeit und Viskosität ermitteln.

Die in den Diagrammen (Figur 2A und 2B) dargestellten Durchflusskurven wurden mit der Messapparatur aus Figur 6 mit hochreinem Wasser für Katheterballone mit unterschiedlicher Bohrungsanzahl gemessen.

### Beispiel 3: Erfolglose Versuche der Herstellung von Katheterballons mit Mikrobohrungen wie in WO 2012/156914 A2 und US 2011/160575 A1 beschrieben

Die Bohrungen in die Katheterballonen mit Durchmessern < 2,5 µm, vorzugsweise von 1,7 µm wurden durch Ionenbestrahlung mittels einem Zyklotron mit anschließendem chemischem Ätzen hergestellt (Figur 7). Die Ionenstrahlen bzw. Ionenpartikel durchdringen die Kunststoffwandung des Ballons und cracken die Moleküle entlang des Durchdringungskanals, so dass durch chemisches Ätzen winzige Kanäle in der Ballonwandung erzeugt werden. Mit diesem Verfahren konnten kleinste Bohrungen von 0,8 µm Durchmesser erzeugt werden.

Gemäß der Anmeldung aus 2012 lassen sich semi-compliant Ballone nicht mit dem "track etching" Verfahren herstellen (s.S. 21 Z 9-12 von WO 2012/156914 A2), das Ionenstrahlbohrverfahren kann also nur bei non-compliant Ballone angewendet werden.

Die Bohrungsdurchmesser lassen sich im **Ionenstrahlverfahren** nicht gezielt vergrößern, da die Ionen eine feste Größe/Durchmesser haben und auch keine Fokussierung mehrerer Ionenpartikel stattfindet, sondern der Katheterballon einem "Bombardement" von einzelnen Partikeln ausgesetzt ist. Zum Anmeldezeitpunkt der WO 2012/156914 A2 konnten im Ionenstrahlverfahren keine Bohrungen in die Ballonkatheter größer als 2,5 µm hergestellt werden (Figur 7).

Weitere Einschränkungen des Ionenstrahl-Bohrungsverfahrens sind: Die Anzahl der Bohrungen lassen sich nur über die Zeitdauer des Ionenbeschusses einstellen, eine singuläre Herstellung einzelner Bohrungen ist nicht möglich. Die Positionierung einzelner Bohrungen auf dem Ballon ist ebenfalls nicht möglich, die Verteilung der Bohrungen ist stochastisch. Lediglich durch Abschattungen des Ionenstrahls lassen sich Bohrungsbereiche auf dem Ballon festlegen. Die Ionenstrahlbohrungen waren damit undefinierbar und eine verlässlich reproduzierbare Wirkstoffabgabe durch serienmäßig gebohrte Ballone nicht möglich.

Der entscheidende Nachteil des Ionenstrahlverfahrens ist aber die anschließende chemische Ätzung zum Öffnen der Mikrobohrungen. Dadurch dringt Säure in den Katheterballon, die sich weder durch die Ballonbohrungen noch durch den Katheterschlauch ausspülen lässt. Durch diese Kontamination des Balloninneren wird der Katheterballon für den Einsatz im menschlichen Körper unbrauchbar.

Aufgrund der genannten Nachteile und Probleme des Ionenstrahl-Bohrverfahrens wurde alternativ das **Laserstrahlbohren** der Katheterballone untersucht, wobei Katheterballone aus PET und Nylon verwendet wurden. Die Untersuchungen hierzu wurden hauptsächlich beim Patentanmelder (Firma MeKo) durchgeführt im Zeitraum von Ende 2010 bis Mitte 2012. Gemäß Zeichnung sollten die Bohrungen 2,0 ± 0,2 µm groß sein (Figur 8).

Mikrobohrungen im Durchmesserbereich von wenigen Mikrometern, zudem in Kunststoffen, lassen sich nur mit ultrakurzen Laserpulsen von kürzer als 100 ps (Pikosekunden), sogenannten **Ultrakurzpulslasern** (UKP-Laser) herstellen. Bei längeren Pulsdauern kommt es durch Wärmeleitung zur Erwärmung bzw. zur Erschmelzung größerer Bohrungsbereiche und damit zu größeren Bohrungsdurchmessern. Aufgrund der extrem kurzen Laserpulse ist der Wärmeeintrag in das Material äußerst gering.

Die Ultrakurzpulslaser müssen eine sehr gute Laserstrahlqualität aufweisen, um den Laserstrahl auf einen winzigen Durchmesser zu fokussieren. Das theoretische physikalische Limit des kleinsten Fokusdurchmessers entspricht der Strahlungswellenlänge, also bei einer Wellenlänge von 1030 nm entsprechend ∼ 1 µm Fokusdurchmesser. Der real erreichbare kleinste Fokusdurchmesser ist jedoch stets deutlich größer aus folgenden Gründen: Bei der Erzeugung von ultrakurzen Pulsdauern sind die Pulsleistungen extrem hoch. Bei hohen Pulsleistungen werden systembedingt höhere Strahl-Schwingungsformen (sogenannte Modenordnungen) angeregt. Laserstrahlen höherer Modenordnung lassen sich schlechter fokussieren.

Zudem hatte zum Anmeldezeitpunkt von WO 2012/156914 A2 die relativ neue Lasertechnologie der ultrakurzen Pulse bzw. die UKP-Laser noch nicht den Entwicklungsstand, um Bohrungen im Bereich von 2 µm und kleiner zu erzeugen, wie im Folgenden erläutert wird.

Die Pulsdauern der leistungsfähigen UKP-Laser lagen im Bereich von Pikosekunden und waren damit zu lang, um Wärmeleitung und Aufschmelzungen zu vermeiden. In Figur 10 links sind daher geschmolzene Materialaufwürfe zu sehen, die erst mit neuerer UKP-Lasertechnik (nach 2012) und deutlich kürzeren Pulsdauern vermieden werden konnten.

Typische leistungsfähige UKP-Laser emittieren bei einer Wellenlänge von 1030/1035 bzw. 1064 nm. Um die Auflösung d.h. die Fokussierbarkeit und die Strahlungsabsorption der zu bearbeitenden Materialien zu verbessern, wird die Strahlungswellenlänge durch spezielle optische Medien verkürzt. Bei dieser Frequenzkonversion durchläuft der Laserstrahl ein optisches Medium (Kristall) und es werden sogenannte höhere Harmonische mit der doppelten (SHG) oder dreifachen (THG) Frequenz bzw. halben (515 nm) oder ein Drittel (343 nm) der eingeleiteten Wellenlänge erzeugt. Einerseits wird die Fokussierbarkeit durch die kürzere Wellenlänge verbessert, andererseits tragen zusätzliche optische Elemente zu einer Strahlverschlechterung/-aufweitung bei. Außerdem ist die Frequenzkonversion mit Leistungsverlusten verbunden.

Die begrenzte Strahlqualität des UKP-Laserstrahls zeigt insbesondere Figur 10 rechts. Um die mittige Bohrung herum sind Materialschädigungen zu erkennen, die aufgrund eines aufgefächerten Laserstrahls entstehen. Aufgrund des aufgefächerten Laserstrahls wurde mit der grünen Wellenlänge 515 nm gebohrt und nicht mit der (besser fokussierbaren) ultravioletten Wellenlänge von 343 nm, da UV-Strahlen Kunststoffverbindungen cracken und das Ballonmaterial schädigen. Die Bereiche um die eigentliche Laserstrahlbohrung herum wurden durch die diffuse UV-Laserstrahlung so geschädigt, dass die Bohrungen bei hohen Ballondrücken aufgerissen sind.

Die Maschinen-/Anlagentechnik und die Prozesstechnik hatte außerdem noch nicht den erforderlichen technologischen Reifegrad. Die Strahlführungs- und Fokussieroptiken wiesen nicht die erforderlichen verzehrungsfreien Optikqualitäten auf. Das Ziel, selbst kleinste Bohrungen von wenigen Mikrometern mittig mit dem Laserstrahl einzustechen und dann auszuschneiden, ließ sich nicht verwirklichen, da die Verfahrgenauigkeit der Bewegungstische auf ± 0,5 µm beschränkt war.

Für die Katheterballone wurde eine mittlere Wandstärke von ~24 µm gemessen (siehe Figur 9). Bei geforderten ~2 µm großen Bohrungen bedeutet dies ein hohes Bohrungsdurchmesser - Wandstärkenverhältnis von über 1:10. Mit anderen Worten: der Laserstrahldurchmesser müsste über eine Wandstärke von 24 µm einen konstanten Durchmesser von 2 µm aufweisen. Die hierfür notwendige Tiefenschärfe im Strahlfokus (Rayleighlänge) war und ist bis heute nicht gegeben.

Auch die Wandstärkenschwankungen auf einem Katheterballon (siehe Figur 9) von ± 2 µm erschweren das Laserbohren im µm-Bereich. Wenn der Bohrungsdurchmesser unabhängig von Wandstärkeschwankungen konstant sein soll, muss ein nahezu paralleler Strahl im Strahlfokus vorliegen, d.h. es ist eine hohe Tiefenschärfe im Fokus gefordert. Die hierzu notwendige Strahlqualität wurde in den Jahren 2010 bis 2012 noch nicht von den Lasersystemen erreicht.

Um dennoch kleinste Bohrungsdurchmesser von 2 µm zu erzielen (siehe Zeichnung Figur 8), wurden in den Jahren 2010 bis 2012 konische Bohrungen in die Ballonwandung lasergebohrt (s.S. 21 Z 9-13 von WO 2012/156914 A2). Die Prozessparameter wurden so eingestellt, dass im Moment der ersten vollständigen Durchdringung der Wandung die Bearbeitung abgebrochen wurde. Bei diesem Verfahren des Laserbohrens von konischen Bohrungen mit Abbruch des Bohrens nach vorbestimmter Zeit führen Schwankungen in der Ballonwanddicke zu deutlich unterschiedlichen Bohrungsdurchmessern. Bei dickerer Wandung wird ggfs. nicht durchgebohrt und bei dünnerer Wandung entsteht eine zu große Bohrung. Die Bohrungsdurchmesser streuten um mehr als 1 µm im Durchmesser, was bei Sollwerten von ~2 µm zu groß ist (siehe Figur 10 und 11).

Figuren 10 und 11 zeigen exemplarisch konische Laserstrahlbohrungen. Der Durchmesser der Bohrungen auf der Ballonaußenseite (Laserstrahl-Eintrittsseite) beträgt ~7 µm und auf der Balloninnenseite (Laserstrahl-Austrittsseite) 1,8 µm bzw. 2,7 µm.

Wie bereits erläutert war die Strahlqualität der UKP-Laser in den Jahren 2011/2012 noch eingeschränkt. In den Figuren 10 und 11 sind auf der Laserstrahl-Eintrittsseite Materialaufwürfe rings um die Bohrung zu sehen. Auf der Laserstrahl-Austrittsseite sind um die Bohrung diffuse Lochmuster zu sehen, was auf eine Auffächerung eines nicht optimal fokussierten Laserstrahls schließen lässt.

Zusammenfassend existierte ein ganzes Bündel von technologischen Beschränkungen wie:
- zu lange Laserstrahlpulsdauern
- zu schlechte Laserstrahlqualität
- zu große Wellenlänge
- zu dicke Wandstärke der Katheterballone mit Wandstärkenschwankungen
- eingeschränkte Anlagentechnik und Optikqualitäten.

Durch die Summe dieser Einschränkungen konnte der in der Patentanmeldung WO 2012/156914 A2 genannte Bohrungs-Durchmesserbereich 0,8 - 5 µm nicht verlässlich und reproduzierbar und nicht für die erfindungsgemäße Bestimmung geeignet durch Laserstrahlbohrungen hergestellt werden. Sämtliche Versuche zur Bestimmung der Wirkstoffabgabe beziehen sich daher in WO 2012/156914 A2 auf Bohrungsdurchmesser ≤ 2,5 µm, vorzugsweise auf Bohrungen mit 1,7 µm Durchmesser (WO 2012/156914 A2 Seite 12 Randziffer 14).

### Beispiel 4: Optimierte Herstellung von Katheterballonen mit Mikrobohrungen 3,0 µm bis 7,5 µm

Gegenüber den Laserstrahlbohrungen von 2010 bis 2012 wurden folgende Verbesserung erreicht:
a) Neuere UKP-Laserstrahlquellen wiesen die erforderlich hohen Strahlleistungen bei **kürzeren Pulsdauern** im Femtosekundenbereich auf. Beim Laserstrahlbohren mit Femtosekunden-Pulsdauern erfolgt nahezu keine Wärmeleitung mehr in die Ballonwandung. Es kommt zu keinen Aufschmelzungen, d.h. Materialaufwürfen auf der Laserstrahleintrittsseite (vergleiche Figur 10/11 mit Figur 12).
b) Leistungsfähige Ultrakurzpulslaser emittieren typischerweise im nahen Infrarot bei einer Wellenlänge von 1030 nm (oder 1064) nm. Für diese Wellenlänge weisen insbesondere transparente Kunststoffe eine geringe Strahlungsabsorption auf. Um eine **höhere Absorption** zu erzielen, erfolgte eine Frequenzkonversion bzw. Frequenzvervielfachung mit speziellen Medien (Kristallen) zu kürzeren Wellenlängen, von 515 nm (532 nm) in den Jahren 2010 bis 2012, auf 343 nm (355 nm).
c) Mit kürzerer Strahlungswellenlänge verbessern sich die Fokussierungseigenschaften, da das physikalische Limit des kleinsten erreichbaren Fokusdurchmessers der Strahlungswellenlänge entspricht. Die kürzere Strahlungswellenlänge von 343 nm erlaubt (gegenüber der zuvor verwandten Wellenlänge von 515 nm) eine **kleinere Strahlfokussierung** um circa 33% entsprechend dem Verhältnis der Strahlungswellenlängen 515 nm zu 343 nm.
d) Sowohl verbesserte UKP-Laser als auch optimierte Optikkomponenten wie die Medien zur Frequenzkonversion führten zu einer besseren Strahlqualität. Die optimierte Laserstrahlqualität ermöglicht einen klar abgegrenzten Laserstrahl **ohne diffuse Laserstrahlrandbereiche.** Hierdurch war es möglich mit der kürzeren Strahlungswellenlänge von 343 nm zu bohren, ohne mit dieser ultravioletten Strahlung eine Schädigung des Ballonkunststoffes zu riskieren.
e) Es werden Katheterballone mit **dünneren Ballonwandungen von 13-18 µm** anstelle von früher 22 - 27 µm verwandt. Die erforderliche Tiefenschärfe, d.h. die Länge konstanten Strahldurchmessers im Fokusbereich ist verringert. Bei geringerer erforderlicher Tiefenschärfe kann mit einer kürzeren Fokussierbrennweite gearbeitet werden, wodurch ein kleinerer Fokusdurchmesser erreicht wird. Ebenso konnte der Laserstrahl vor Eintritt in die Fokussierungskomponenten stärker aufgeweitet werden, was zu einem kleineren Fokusdurchmesser führt.
f) Die **Wanddickentoleranz** der Ballonwandungen ist verbessert, womit ein stabilerer Laserstrahlbohrprozess mit eingeschränkten Bohrungstoleranzen einhergeht.
g) Die **Maschinen-/Anlagentechnik** wurde verbessert mit einer höheren Verfahrgenauigkeit der x,y-Bearbeitungstische. Damit können die Mikrobohrungen im Bereich 3,0 µm bis 7,5 µm lasergeschnitten werden, d.h. im Bohrungsmittelpunkt wird eingestochen und anschließend die Bohrung geschnitten. Dies erlaubt eine flexible Einstellung der Bohrungsdurchmesser durch Anpassung der Radiuskorrektur beim Laserschneiden.
h) Auch die Haltevorrichtungen bzw. Vorrichtungen zur präzisen Positionierung der Ballone unterhalb des fokussierten Laserstrahls wurden verbessert, insbesondere die präzise Einhaltung der Fokuslage des Laserstrahlstrahls in der Ballonwandung.

Die Verfahrensentwicklung zum Laserstrahlbohren hat sich neben der Erzielung von Bohrungen im relevanten Durchmesserbereich von 3,0 bis 7,5 µm insbesondere auf die Einhaltung geringer Durchmessertoleranzen konzentriert. Durch Verfahrensoptimierung und Prozessstabilisierung des Laserstrahlbohrens werden Toleranzen von weniger als 5 % erzielt. Bohrungsdurchmesser von 5 µm werden mit einer Genauigkeit von ± 0,2 µm hergestellt, entsprechend ± 4 % Durchmessertoleranz (siehe Figur 12).

Durch die Vielzahl der Verfahrens- und Prozessoptimierungen konnte ein stabiles und reproduzierbares Laserstrahlbohren von Katheterballonen mit Bohrungen von 3,0 µm bis 7,5 µm sichergestellt werden. Nur mit reproduzierbar gebohrten Katheterballonen kann eine Bestimmung der Flüssigkeitsabgabe mittels Druckmessung erfolgen, wie in den Ansprüchen 1 bis 8 der Patentanmeldung beansprucht.

### Bezugszeichen

- 1: Inflationsvorrichtung
- 2: Inflator (Pump Unit)
- 3: Drucksensor
- 4: Zeitgeber
- 5: Recheneinheit
- 6: Anzeigeeinheit / Display
- 7: Drossel

- 10: Katheterballon
- 11: Mikrobohrungen
- 15: Metallnetz

- 20: Wirkstoffflüssigkeit
- 21: Wirkstoffdispersion
- 22: Wirkstoffdispersion von Mikrospheren
- 23: Wirkstoffdispersion von Nanospheren

- 30: Korrelationskurve

- 40: (schlauchartiges) Zuführsystem

- 50: Wirkstoffflüssigkeitsabgabeeinheit

- 60: Datenübertragungseinheit
- 61: Lesegerät

## Patentansprüche

1. Eine Vorrichtung (1) zur dosierten Freisetzung einer Wirkstoffflüssigkeit (20) über eine Wirkstoffflüssigkeitsabgabeeinheit (50) im Körperinneren umfassend
- eine Pumpeinheit (2) mit angeschlossenem schlauchartigen Zuführsystem (40),
- mindestens eine Drossel (7) zur Begrenzung des Volumenstroms verbunden mit einem Druckaufbau im Zuführsystem,
- einen Drucksensor (3) zur Bestimmung des durch die Pumpeinheit erzeugten Drucks,
- einen Zeitgeber (4), der ausgelegt ist, einen Zeitraum, in dem die Wirkstoffflüssigkeit freigesetzt wird, zu erfassen,
- eine Recheneinheit (5), die konfiguriert ist, das von der mindestens einen Drossel (7) freigesetzte Volumen an Wirkstoffflüssigkeit (20) aus den vom Drucksensor (3) empfangenen Druckwerten und den vom Zeitgeber (4) empfangenen Zeitinkrementen mittels einer gespeicherten Korrelationskurve (30) für diesen Drosseltyp (7) und für diese Wirkstoffflüssigkeit (20) zu berechnen, und
- eine Anzeigeeinheit (6), die konfiguriert ist, die vom Drucksensor (3) empfangenen Daten und das von der Recheneinheit (5) berechnete freigesetzte Volumen an Wirkstoffflüssigkeit (20) anzuzeigen.

2. Die Vorrichtung (1) gemäß Anspruch 1, wobei die mindestens eine Drossel (7) unmittelbar vor der Wirkstoffflüssigkeitsabgabeeinheit (50) angeordnet ist, und wobei die mindestens eine Drossel (7) so als Querschnittsverengung im Zuführsystem (40) ausgestaltet ist, dass sie zu einem Druckanstieg vor der Drossel (7) im Zuführsystem (40) führt.

3. Die Vorrichtung (1) gemäß Anspruch 1 oder 2, wobei die mindestens eine Drossel (7) als Querschnittverengung in Form einer Lochblende, einem Spalt, einer Düse, einer Kapillare oder mehreren Kapillarnadeln oder einer anderen beliebigen Form besteht.

4. Die Vorrichtung (1) gemäß einem der Ansprüche 1 bis 3, wobei für die jeweilige Drossel bzw. für die Querschnittsverengung und die Viskosität der Flüssigkeit eine Korrelationskurve (30) für die Flüssigkeitsrate in Abhängigkeit vom Flüssigkeitsdruck gemessen und in der Recheneinheit (5) als Datenfile hinterlegt ist.

5. Die Vorrichtung (1) gemäß einem der Ansprüche 2 bis 4, wobei die mindestens eine Drossel (7) so ausgestaltet ist, dass hinter der mindestens einen Drossel (7) ein vernachlässigbarer kleiner Druck im Zuführsystem (40) besteht und die Wirkstoffflüssigkeit (20) über eine Wirkstoffflüssigkeitsabgabeeinheit (50) an die Wirkstelle im Körperinneren drucklos abgegeben wird.

6. Die Vorrichtung (1) gemäß einem der Ansprüche 1 bis 5, wobei die Recheneinheit (5) konfiguriert ist, um das von der Wirkstoffflüssigkeitsabgabeeinheit (50) freigesetzte Volumen an Wirkstoffflüssigkeit (20) über Flüssigkeitsdruck, Zeitinkrement und Viskosität der Wirkstoffflüssigkeit (20) mittels der Korrelationskurve (30) zu bestimmen.

7. Die Vorrichtung (1) gemäß einem der Ansprüche 1 bis 6, wobei die Anzeigeeinheit (6) eine Digitalanzeige umfasst, die den Flüssigkeitsdruck, die Inflationszeit des Katheterballons (10) und das abgegebene Volumen an Wirkstoffflüssigkeit (20) anzeigt.

8. Die Vorrichtung (1) gemäß Anspruch 1, wobei die Wirkstoffflüssigkeitsabgabeeinheit (50) ein Ballonkatheter mit einem **mikrogebohrten non-compliant Katheterballon** (10) ist, der zur Aufnahme einer Wirkstoffflüssigkeit (20) ausgestaltet ist, welche durch die Mikrobohrungen (11) applizierbar ist, und wobei es sich bei der mindestens einen Drossel (7) um Mikrobohrungen (11) in dem mikrogebohrten non-compliant Katheterballon (10) handelt und die Mikrobohrungen (11) einen Bohrungsdurchmesser im Bereich von 3,0 µm bis 7,5 µm mit einer Toleranz von maximal ±0,2 µm aufweisen.

9. Die Vorrichtung (1) gemäß Anspruch 8, wobei die Anzahl der Mikrobohrungen (11) in dem non-compliant Katheterballon (10) von 0,3 bis 4,0 Mikrobohrungen pro mm², bevorzugt 1,0 - 3,0 Mikrobohrungen pro mm², weiter bevorzugt 1,5 bis 2,5 Mikrobohrungen pro mm² Ballonoberfläche beträgt.

10. Die Vorrichtung (1) gemäß Anspruch 8 oder 9, wobei der Ballonkatheter aus einem mikrogebohrten Ballonkatheter mit integrierter **Lithotripsie** Vorrichtung zur Erzeugung von Schallwellen besteht oder aus einem Katheter mit als Drossel dienenden **Injektionsnadeln** zur direkten Wirkstoffabgabe ins Gewebe.

11. Die Vorrichtung (1) gemäß einem der Ansprüche 8 bis 10, wobei die Mikrobohrungen (11) im Katheterballon (10) einen Bohrungsdurchmesser im Bereich von 3,0 µm bis 7,5 µm oder 3,0 µm bis 7,5 µm, bevorzugt von 3,5 µm bis 7,5 µm oder 3,5 µm bis 7,0 µm, bevorzugt von 3,5 µm bis 6,5 µm, bevorzugt von 4,0 µm bis 7,0 µm, bevorzugt von 4,0 µm bis 6,5 µm, weiter bevorzugt von 4,0 µm bis 6,0 µm aufweisen und die Mikrobohrungen (11) im Katheterballon (10) einen Bohrungsdurchmesser mit einer Toleranz von 5% oder weniger oder eine Genauigkeit von maximal ±0,2 µm aufweisen.

12. Die Vorrichtung (1) gemäß einem der Ansprüche 8 bis 11, wobei der Katheterballon (10) mit einem Vlies oder einem Filz oder einem Gewebe oder einem Metallnetz (15) überzogen ist und/oder wobei die Mikrobohrungen (11) in dem Katheterballon (10) bei einem Balloninnendruck von 0,2 MPa bis 3 MPa zur Applikation der Wirkstoffflüssigkeit (20) geeignet sind.

13. Die Vorrichtung (1) gemäß einem der Ansprüche 8 bis 12, wobei es sich bei der Wirkstoffflüssigkeit (20) um eine Wirkstofflösung oder eine Wirkstoffdispersion handelt.

14. Die Vorrichtung (1) gemäß einem der Ansprüche 8 bis 13, wobei die Vorrichtung (1) zur dosierten Freisetzung einer Wirkstoffdispersion von Mikrospheren (22)
- einen Inflator (2),
- einen Drucksensor (3) zur Bestimmung des durch den Inflator (2) erzeugten Drucks,
- einen Zeitgeber (4), der ausgelegt ist, einen Zeitraum, in dem die Wirkstoffdispersion von Mikrospheren (22) freigesetzt wird, zu erfassen,
- eine Recheneinheit (5), die konfiguriert ist, das vom Katheterballon (10) durch die Mikrobohrungen (11) freigesetzte Volumen an Wirkstoffdispersion von Mikrospheren (22) aus den vom Drucksensor (3) empfangenen Druckwerten und den vom Zeitgeber (4) empfangenen Zeitinkrementen mittels einer gespeicherten Korrelationskurve (30) für diesen Katheterballon (10) und für diese Wirkstoffdispersion von Mikrospheren (22) zu berechnen, und
- eine Anzeigeeinheit (6) umfasst, die konfiguriert ist, die vom Drucksensor (3) empfangenen Daten und das von der Recheneinheit (5) berechnete freigesetzte Volumen an Wirkstoffdispersion von Mikrospheren (22) anzuzeigen,
wobei der Katheterballon (10) Mikrobohrungen (11) mit einem Bohrungsdurchmesser im Bereich von 3,5 µm bis 7,5 µm oder 4,0 µm bis 7,5 µm, bevorzugt von 4,5 µm bis 7,5 µm, weiter bevorzugt von 5,0 µm bis 7,5 µm, weiter bevorzugt von 5,5 µm bis 7,5 µm aufweist.

15. Die Vorrichtung (1) gemäß einem der Ansprüche 1 bis 14, ferner umfassend eine Datenübertragungseinheit (60) mit Lesegerät (61) zur Identifizierung der verwendeten Wirkstoffflüssigkeit (20) und deren Viskosität und des verwendeten Zuführsystems mit Drossel, wobei die Datenübertragungseinheit konfiguriert ist, die Korrelationskurve (30) anhand der identifizierten Wirkstoffflüssigkeit (20) und der identifizierten Drossel (7) auszuwählen.
